# EUROPEAN PATENT SPECIFICATION

(11) **EP 1 648 919 B1**
(45) Date of publication and mention of the grant of the patent: **17.11.2010**
(21) Application number: 04779108.2
(22) Date of filing: 22.07.2004
(51) Int. Cl.: G01N 33/543, G01N 33/53, G01N 33/569, G01N 33/50

(54) **METHODS FOR DETECTING ACTIVATION OF T-CELLS BY MHC BINDING PEPTIDES**
VERFAHREN ZUM NACHWEIS DER AKTIVIERUNG VON T-ZELLEN DURCH MHC-BINDENDE PEPTIDE
METHODES DE DETECTION DE L'ACTIVATION DES LYMPHOCYTES T PAR DES PEPTIDES DE LIAISON AU CMH

(30) Priority: 22.07.2003 US 489359 P
(43) Date of publication of application: 26.04.2006
(73) Proprietor: Beckman Coulter, Inc., Brea, 92821 CA (US)
(72) Inventor: CHANG, Jennie Chyan Chuu, San Marcos, CA 92069 (US); KASEY, Suha, San Marcos, CA 92078 (US); CREBASSA, Veronique Trigueros, F-31300 Toulouse (FR); MONTERO-JULIAN, Felix A., F-13276 Marseille, Cedex 9 (FR)
(74) Representative: Cornish, Kristina Victoria Joy
(86) International application number: PCT/US2004/023898
(87) International publication number: WO 2005/010026

(56) References cited:
- WO-A-01/90747
- WO-A-2004/094458
- US-A1- 2003 044 389
- US-A1- 2003 073 102
- HUGUES S ET AL: "Generation and use of alternative multimers of peptide/MHC complexes" JOURNAL OF IMMUNOLOGICAL METHODS, ELSEVIER, AMSTERDAM, NL, vol. 268, no. 1, 1 October 2002 (2002-10-01), pages 83-92, XP004379139 ISSN: 0022-1759
- ABASTADO ET AL.: 'Dimerization of soluble major histocompatibility complex-peptide complexes is sufficient for activation of T cell hybridoma and induction of unresponsiveness' J. EXP. MED. vol. 182, August 1995, pages 439 - 447, XP009005734
- HARLOW E. ET AL.: 'Antibodies, A Laborator Manual', 1988, COLD SPRING HARBOR LAB, USA pages 578 - 581, XP008084632
- RAMMENSEE ET AL.: 'MHC Ligands and peptide motifs: first listing' LANDES BIOSCIENCE 1997, AUSTIN, TX, USA, pages 18 - 19, XP008084798

## Description

### FIELD OF THE INVENTION

This invention relates generally to the field of immunoassays, especially using immunoassays to detect and measure activation of T-cells with a recombinant HLA monomer in a solid phase format.

### BACKGROUND OF THE INVENTION

The Class I histocompatibility ternary complex consists of three parts associated by noncovalent bonds. A transmembrane protein, called the MHC heavy chain is mostly exposed at the cell surface. The cell surface domains of the MHC monomer contain two segments of alpha helix that form two ridges with a groove between them. A short peptide binds noncovalently ("fits") into this groove between the two alpha helices, and a molecule of beta-2 microglobulin binds noncovalently along side the outer two domains of the MHC monomer, forming a ternary complex. Peptides that bind noncovalently to one MHC subtype monomer usually will not bind to another subtype. However, all bind with the same type of beta-2 microglobulin. MHC monomers are synthesized and displayed by most of the cells of the body.

In humans, MHC molecules are referred to as HLA molecules. Humans synthesize three different sub-types of MHC molecules designated HLA-A, HLA-B and HLA-C, differing only in the heavy chains.

The MHC monomer works coordinately with a specialized type of T-cell (the cytotoxic T-cell) to rid the body of "nonself' or foreign viral proteins. The antigen receptor on T-cells recognizes an epitope that is a mosaic of the bound peptide and portions of the alpha helices of the monomer making up the groove flanking it. Following generation of peptide fragments by cleavage of a foreign protein, the presentation of peptide fragments by the MHC monomer in the ternary complex allows for MHC-restricted cytotoxic T-cells to survey cells for the expression of "nonself" or foreign viral proteins. A functional T-cell will exhibit a cytotoxic immune response upon recognition of an MHC monomer containing bound antigenic peptide for which the T-cell is specific.

In the performance of these functions in humans, HLA-A, B, and C monomers interact with a multitude of peptides of about 8 to about 10, possibly about 8 to about 11, or about 8 to about 12 amino acids in length. Only certain peptides bind into the binding pocket in the monomer of each HLA sub-type as the monomer folds, although certain subtypes cross-react. By 1995, complete coding region sequences had been determined for each of 43 HLA-A, 89 HLA-B and 11 HLA-C alleles (P. Parham et al., Immunology Review 143:141-180, 1995).

Class II histocompatibility molecules consist of two transmembrane polypeptides that interact to form a groove at their outer end which, like the groove in class I molecules, non-covalently associates with an antigenic peptide. However, the antigenic peptides bound to class II molecules are derived from antigens that the cell has taken in from its surroundings. In addition, peptides that bind to class II histocompatibility molecules are 15 to about 30 amino acids in length. Only cells, such as macrophages and B lymphocytes, that specialize in taking up antigen from extracellular fluids, express class II molecules.

It has long been thought that discovery of which antigen fragments will be recognized by class I MHC-restricted T-cells can lead to development of effective vaccines against cancer and viral infections. A number of approaches have been developed wherein algorithms are used to predict the amino acid sequence of HLA A, B, or C-binding peptides and several are available on the internet. For example, U.S. Patent No. 6,037,135 describes a matrix-based algorithm that ranks peptides for likelihood of binding to any given HLA-A allele. Similarly, most prediction methods are limited to a set of alleles. Consequently, the predicted peptides may not bind to MHC monomers from a whole population of patients and thus may not be globally effective.

Another approach to identifying MHC-binding peptides uses a competition-based binding assay. All competition assays yield a comparison of binding affinities of different peptides. However, such assays do not yield an absolute dissociation constant since the result is dependent on the reference peptide used.

Still another approach used for determining MHC-binding peptides is the classical reconstitution assay, in which cells expressing an appropriate MHC allele are "stripped" of a native binding peptide by incubating at pH 2-3 for a short period of time. Then, to determine the binding affinity of a putative MHC-binding peptide for the same MHC allele, the MHC monomer stripped of its binding peptide is combined in solution with the putative MHC-binding peptide, beta2-microglobulin and a conformation-dependent monoclonal antibody. The difference in fluorescence intensity determined between cells incubated with and without the test binding peptide after labeling, for example, either directly with the labeled monoclonal antibody or a fluorescence-labeled secondary antibody, can be used to determine binding of the test peptide. However, soluble MHC monomers stripped at low pH aggregate immediately, making their use in high through-put assays difficult and impractical.

There are currently a series of *in vitro* assays for cell mediated immunity which use cells from the host both as the substrate cell that initiates or stimulates the reaction for which the assay has been developed and as the target to assess cell mediated immunity. These *in vitro* assays include the cytotoxic T lymphocyte assay; cytokine production assay (Salio et al., Eur. J. Immunol. 33: 1052-1062, 2003 and Carrabba et al., Cancer Research 63: 1560-1567, 2003) lymphoproliferative assays, e.g., tritiated thymidine incorporation; the protein kinase assays, the ion transport assay and the lymphocyte migration inhibition function assay (Hickling, J. K. et al, J. Virol., 61: 3463 (1987); Hengel, H. et al, J. Immunol., 139: 4196 (1987); Thorley-Lawson, D. A. et al, Proc. Natl. Acad. Sci. USA, 84: 5384 (1987); Kadival, G. J. et al, J. Immunol., 139: 2447 (1987); Samuelson, L. E. et el, J. Immunol., 139: 2708 (1987); Cason, J. et al, J. Immunol. Meth., 102: 109 (1987); and Tsein, R. J. et al, Nature, 293: 68 (1982)). These assays are disadvantageous in that they may lack true specificity for cell mediated immunity activity, they require antigen processing and presentation by an APC of the same MHC type, they are slow (sometimes lasting several days), and some are subjective and/or require the use of radioisotopes.

Yet another approach to identifying MHC class I-binding peptides utilizes formation of MHC tetramers, which are complexes of four MHC monomers with streptavidin, a molecule having tetrameric binding sites for biotin, to which is bound a fluorochrome, e.g., phycoerythrin. For class I monomers, soluble subunits of β2-microglobulin, the peptide fragment containing a putative T-cell epitope, and a MHC heavy chain corresponding to the predicted MHC subtype of the peptide fragment of interest, are obtained, for example, by recombinant expression of polynucleotides encoding these polypeptides in host-cells. Each of the four monomers contained in the MHC tetramer is produced as a monomer by refolding these soluble subunits under conditions that favor assembly of the soluble units into reconstituted monomers, each containing a beta2-microglobulin, a peptide fragment, and the corresponding MHC- heavy chain. An MHC tetramer is constructed from the monomers by biotinylation of the monomers and subsequent contact of the biotinylated reconstituted monomers with fluorochrome-labeled streptavidin. When contacted with a diverse population of T-cells, such as is contained in a sample of the peripheral blood lymphocytes (PBLs) of a subject, those tetramers containing reconstituted monomers that are recognized by a T-cell in the sample will bind to the matched T-cell. Contents of the reaction is analyzed using fluorescence flow cytometry, to determine, quantify and/or isolate those T-cells having a MHC tetramer bound thereto. MHC-binding peptides are found in those tetramers that are recognized by T-cells. (See U. S. Patent No. 5,635,363).

At least one other test is required to determine whether a test peptide recognized by a T-cell by the MHC tetramer assay will activate the T-cell to generate an immune response. The enzyme-linked immunospot (ELISpot) assay has been adapted for the detection of individual cells secreting specific cytokines or other antigens by attachment of a monoclonal antibody specific for a cytokine to a coating on a microplate. Cells stimulated by an antigen are contacted with the immobilized antibody. After washing away cells and any unbound substances, a tagged polyclonal antibody specific for the same cytokine is added to the wells. Following a wash, a colorant that binds to the tagged antibody is added such that a blue-black colored precipitate (or spot) forms at the sites of cytokine localization. The spots can be counted manually or with automated ELISpot reader system to quantitate the response. A final confirmation of T-cell activation by the test peptide may require in vivo testing. Thus, the route to final confirmation of the efficacy of a MHC-binding peptide is expensive and time consuming.

It has been shown previously that T-cells, expressing the appropriate TCR, either CD4 or CD8, can be activated by a peptide-HLA monomer complex. T-cell activation can be detected measuring either the release of secreted factors, mobilization of intracellular calcium or by measuring T-cell proliferation (³H Thymidine, CFSE (carboxyfluorescein diacetate succinimidyl ester)) or cell surface receptor (e. g. CD25, CD69 etc. ) expression. Interferon gamma represents the most common measured released soluble factor after T-cell activation. Several techniques exist in the art for detection of soluble factors: the most common are ELISA, ELISPOT and detection of intracellular cytokines by flow cytometry or other methods. However, these techniques have some limitations. Detection of peptide-activation and IFN gamma production by ELISPOT can induce a bias because peptides can bind to any of the six alleles expressed by the human cells presenting the peptide. ELISAs, on the other hand, are not very sensitive, thus limiting their use.

WO 01/90747 describes methods for identifying compounds that modulate an immune complex that includes a T cell receptor and a major histocompatibility complex antigen.

US 2003/0044389 relates to methods of profiling cells.

Thus, there is still a need in the art for new and better systems and methods for preliminary assays identifying peptides that can functionally activate T-cells and for quantitating activated T-cells, for example in a sample containing a mixture of T-cells, such as a patient sample.

### SUMMARY OF THE INVENTION

The invention is as set out in the claims.

In one embodiment the invention provides a method of identifying a peptide capable of activating a peptide-specific T-cell, said method comprising: a) incubating under suitable conditions peptide-specific T-cells and a solid surface having attached thereto: 1) BSA-biotin-avidin coated onto the solid surface; 2) a plurality of biotinylated MHC monomers or modified biotinylated MHC monomers having a bound candidate MHC-binding peptide wherein the biotinylated MHC monomers or modified biotinylated MHC monomers are bound via the biotin to the avidin; and 3) a biotinylated anti-TNF alpha or anti-IFN gamma antibody that forms an antibody-TNF alpha or -IFN gamma complex upon activation of the peptide-specific T-cells by the biotinylated MHC monomer - MHC-binding peptide complex; and b) contacting the antibody-TNF alpha or IFN gamma complex obtained from a) with a second antibody specific for TNF alpha or IFN gamma and wherein the second antibody is tagged with a detectable label so as to allow formation of an antibody-TNF alpha or -IFN gamma-antibody complex; and c) detecting a signal produced by the detectable label, wherein the detecting indicates activation of the peptide-specific T-cells and wherein the activation of the peptide-specific T cells indicates that the bound candidate MHC-binding peptide is capable of activating the peptide-specific T-cells.

In yet another embodiment, the invention provides kits useful in practice of the invention methods. The invention kits comprise a solid surface, wherein the surface has attached thereto a kit comprising: a solid surface, wherein the surface has attached thereto: a) BSA-biotin-avidin; b) one or more biotinylated MHC monomers, modified biotinylated MHC monomers or biotinylated multimers thereof wherein the monomers contain a suitable MHC-binding peptide and wherein the biotinylated MHC monomers, modified biotinylated MHC monomers or biotinylated multimers thereof are bound via the biotin to the avidin; and c) a biotinylated anti-TNF alpha or anti-IFN gamma antibody.

### BRIEF DESCRIPTION OF THE FIGURES

**Figs. 1A-1C** schematically depict the invention methods for detection and measuring soluble factor released from T-cells upon activation.

**Fig. 2** is a graph showing detection of biotinylated-anti-TNF alpha antibody in monomer coated plates.

**Fig. 3** is a graph showing recognition of the TNF in monomer-coated plates and in anti-TNF coated plates using an ELISA format.

**Fig. 4** is a graph showing that production of TNF alpha by the cells stimulated by monomers A2/CMV was a function of the number of cells and the concentration of the monomer used as measured by ELISA.

**Fig. 5** is a graph showing production of Interferon gamma by the cells stimulated by monomers A2/CMV in Fig. 4 above.

**Fig. 6** is a graph showing production of TNF alpha by cells (10,000 cells/well) stimulated by monomers A2/CMV as measured by the invention cytokine capture assay.

**Fig. 7** is a graph showing a comparison of TNF alpha production after monomer-T-cell activation as measured using ELISA and the new capture assay method.

**Figs. 8A-D** are a series of density plots showing the percentage of CD25+ CD8+ T-cells for positive control (Fig. 8A); negative control (Fig. 8B); 10X peptide exchanged (Fig. 8C); and 100X peptide exchanged monomers (Fig. 8D) as determined using the invention methods

**Figs. 9A-D** are a series of density plots showing the percentage of A2-CMV+ CD8+ T-cells for positive control (Fig. 9A); negative control (Fig. 9B); 10X peptide exchanged (Fig. 9C); and 100X peptide exchanged monomers (Fig. 9D) as determined using the invention methods.

**Figs. 10A-D** are a series of density plots showing the percentage of activated (CD25+) CD8+ from PBMC in wells coated with positive control (Fig. 10A); negative control (Fig. 10B); 10X peptide exchanged (Fig. 10C); and 100X peptide exchanged monomers (Fig. 10D) as determined using the invention methods.

**Figs. 11A-D** are a series of density plots showing the percentage of A2-CMV+ CD8+ T-cells from PBMC in wells coated with positive control (Fig. 11A); negative control (Fig. 11B); 10X peptide exchanged (Fig. 11C); and 100X peptide exchanged monomers (Fig. 11D) as determined using the invention methods.

**Fig. 12** is a flow chart describing the Tetramer-Intracellular cytokine (ICC) assay.

**Figs. 13A-B** are density plots showing the percentages of A2-CMV tetramers+ IFNγ-and A2-CMV Tetramer+ and IFNγ+ cells from whole blood and PBMC of two donors in Tetramer-ICC Assay.

### DETAILED DESCRIPTION OF THE INVENTION

In epitope discovery, one of the major questions is how to determine easily if a given peptide-MHC complex represents an epitope recognized by peptide-specific T-cells in patient samples. The present invention is based on the discovery that T-cells can be specifically activated with a recombinant HLA-Class I or Class II monomer or multimer in a microtiter plate format while also determining the degree of antigen-specific T-cell activation by measuring one or more released soluble T-cell activation factors selected from cytokines, chemokines, apoptotic factors, and the like, by antibody capturing released factors in the same plate.

As used herein, the terms "MHC monomer" and "HLA monomer" refer to a Class I MHC heavy chain is assembled into a ternary complex with an appropriate MHC-binding or HLA-binding peptide and beta-2 microglobulin (beta-2m) or that maintains the ability to assemble under renaturing conditions. The terms "MHC monomer" and HLA monomer" are also used to refer to the denatured form of the monomer that results from subjecting the ternary complex to denaturing conditions, causing the monomer to unfold and dissociate from an MHC-binding peptide.

As used herein, the terms "modified MHC monomer" and "modified HLA monomer" refer to class I monomers as described above, but which have been engineered to introduce modifications as described below. These terms also encompass functional fragments of the MHC monomer that maintain the ability to assemble into a ternary complex with an appropriate MHC-binding or HLA-binding peptide and beta-2 microglobulin under renaturing conditions and to dissociate under denaturing conditions. For example, in a functional fragment the class I heavy chain can comprise only the α₁, α₂, α₃, domains, or only α₁, α₂ domains, i.e., the cell surface domains, that participate in formation of the ternary complex. In another embodiment, the class I heavy chain molecules in a modified MHC monomers, or functional fragments thereof, can be contained in a fusion protein or "single chain" molecule and may further include an amino acid sequence functioning as a linker between cell surface domains of the heavy chain, as a detectable marker, or as a ligand to attach the molecule to a solid surface that is coated with a second ligand with which the ligand in the fusion protein reacts. Moreover the terms "modified MHC monomer" and "modified HLA monomer" are intended to encompass chimera containing domains of class I heavy chain molecules from more than one species or from more than one class I subclass. For example, a chimera can be prepared by substitution of a mouse beta-2m for human beta-2m in a human HLA-A2 monomer.

### Preparation of monomers

The Class I MHC in humans is located on chromosome 6 and has three loci, HLA-, HLA-B, and HLA-C. The first two loci have a large number of alleles encoding alloantigens. These are found to consist of a 44 Kd heavy chain subunit and a 12 Kd beta-2 -microglobulin subunit which is common to all antigenic specificities. For example, soluble HLA-A2 can be purified after papain digestion of plasma membranes from the homozygous human lymphoblastoid cell line J-Y as described by Turner, M. J. et al., J. Biol. Chem. (1977) 252:7555-7567. Papain cleaves the 44 Kd heavy chain close to the transmembrane region, yielding a molecule comprised of α₁, α₂, α₃ domains and beta-2 microglobulin.

The MHC monomers can be isolated from appropriate cells or can be recombinantly produced, for example as described by Paul et al, Fundamental Immunology, 2d Ed., W. E. Paul, ed., Ravens Press N.Y. 1989, Chapters 16-18) and readily modified, as described below.

The term "isolated" as applied to MHC monomers herein refers to an MHC glycoprotein heavy chain of MHC class I, which is in other than its native state, for example, not associated with the cell membrane of a cell that normally expresses MHC. This term embraces a full length subunit chain, as well as a functional fragment of the MHC heavy chain. A functional fragment is one comprising an antigen binding site and sequences necessary for recognition by the appropriate T-cell receptor. It typically comprises at least about 60-80%, typically 90-95% of the sequence of the full-length chain. As described herein, the "isolated" MHC subunit component may be recombinantly produced or solubilized from the appropriate cell source.

It is well known that native forms of "mature" MHC glycoprotein monomers will vary somewhat in length because of deletions, substitutions, and insertions or additions of one or more amino acids in the sequences. Thus, the heavy chain in MHC monomers are subject to substantial natural modification, yet are still capable of retaining their functions. Modified protein chains can also be readily designed and manufactured utilizing various recombinant DNA techniques well known to those skilled in the art and described in detail, below. For example, the chains can vary from the naturally occurring sequence at the primary structure level by amino acid substitutions, additions, deletions, and the like. These modifications can be used in a number of combinations to produce the final modified protein chain.

In general, modifications of the genes encoding the MHC monomer may be readily accomplished by a variety of well-known techniques, such as site-directed mutagenesis. The effect of any particular modification can be evaluated by routine screening in a suitable assay for the desired characteristic. For instance, a change in the immunological character of the subunit can be detected by competitive immunoassay with an appropriate antibody. The effect of a modification on the ability of the monomer to activate T-cells can be tested using standard *in vitro* cellular assays or the methods described in the example section, below. Modifications of other properties such as redox or thermal stability, hydrophobicity, susceptibility to proteolysis, or the tendency to aggregate are all assayed according to standard techniques.

Amino acid sequence modification to subunits of MHC monomers can be prepared with various objectives in mind, including increasing the affinity of the subunit for antigenic peptides and/or T-cell receptors, facilitating the stability, purification and preparation of the subunits. The monomers may also be modified to modify plasma half life, improve therapeutic efficacy, or to lessen the severity or occurrence of side effects during therapeutic use of complexes of the present invention. The amino acid sequence modifications of the subunits are usually predetermined variants not found in nature or naturally occurring alleles. The variants typically exhibit the same biological activity (for example, MHC-peptide binding) as the naturally occurring analogue.

Insertional modifications are those in which one or more amino acid residues are introduced into a predetermined site in the subunit of the MHC monomer and which displace the preexisting residues. For instance, insertional modifications can be fusions of heterologous proteins or polypeptides to the amino or carboxyl terminus of the subunits.

Other modifications, include fusions of the heavy chain with a heterologous signal sequence and fusions of the heavy chain to polypeptides having enhanced plasma half life (ordinarily>about 20 hours) such as immunoglobulin chains or fragments thereof as is known in the art.

Substitutional modifications are those in which at least one residue has been removed and a different residue inserted in its place. Nonnatural amino acid (i.e., amino acids not normally found in native proteins), as well as isosteric analogs (amino acid or otherwise) are also suitable for use in this invention.

Substantial changes in function or immunological identity are made by selecting substituting residues that differ in their effect on maintaining the structure of the polypeptide backbone (e.g., as a sheet or helical conformation), the charge or hydrophobicity of the molecule at the target site, or the bulk of the side chain. The substitutions which in general are expected to produce the greatest changes in function will be those in which (a) a hydrophilic residue, e.g., serine or threonine, is substituted for (or by) a hydrophobic residue, e.g. leucine, isoleucine, phenylalanine, valine or alanine; (b) a cysteine or proline is substituted for (or by) any other residue; (c) a residue having an electropositive side chain, e.g., lysine, arginine, or histidine, is substituted for (or by) an electronegative residue, e.g., glutamine or aspartine; or (d) a residue having a bulky side chain, e.g., phenylalanine, is substituted for (or by) one not having a side chain, e.g., glycine.

Substitutional modifications of the subunits also include those where functionally homologous (having at least about 70% homology) domains of other proteins are substituted by routine methods for one or more of the MHC subunit domains. Particularly preferred proteins for this purpose are domains from other species, such as murine species.

Another class of modifications are deletional modifications. Deletions are characterized by the removal of one or more amino acid residues from the heavy chain sequence. Typically, the transmembrane and cytoplasmic domains are deleted. Deletions of cysteine or other labile residues also may be desirable, for example in increasing the oxidative stability of the MHC complex. Deletion or substitutions of potential proteolysis sites, e.g., ArgArg, is accomplished by deleting one of the basic residues or substituting one by glutaminyl or histidyl residues.

A preferred class of substitutional or deletional modifications comprises those involving the transmembrane region of the subunit. Transmembrane regions of MHC heavy chain are highly hydrophobic or lipophilic domains that are the proper size to span the lipid bilayer of the cellular membrane. They are believed to anchor the MHC molecule in the cell membrane. Inactivation of the transmembrane domain, typically by deletion or substitution of transmembrane domain hydroxylation residues, will facilitate recovery and formulation by reducing its cellular or membrane lipid affinity and improving its aqueous solubility. Alternatively, the transmembrane and cytoplasmic domains can be deleted to avoid the introduction of potentially immunogenic epitopes. Inactivation of the membrane binding function is accomplished by deletion of sufficient residues to produce a substantially hydrophilic hydropathy profile at this site or by substitution with heterologous residues which accomplish the same result.

A principal advantage of the transmembrane-inactivated MHC monomer heavy chain is that it may be secreted into the culture medium of recombinant hosts. This variant is soluble in body fluids such as blood and does not have an appreciable affinity for cell membrane lipids, thus considerably simplifying its recovery from recombinant-cell culture. Typically, modified MHC monomers of this invention will not have a functional transmembrane domain in the heavy chain and preferably will not have a functional cytoplasmic sequence. Such modified MHC monomers will have a heavy chain consisting essentially of the effective portion of the extracellular domain of the heavy chain. In some circumstances, the heavy chain comprises sequences from the transmembrane region (up to about 10 amino acids), so long as solubility is not significantly affected.

For example, the transmembrane domain may be substituted by any amino acid sequence, e.g., a random or predetermined sequence of about 5 to 50 serine, threonine, lysine, arginine, glutamine, aspartic acid and like hydrophilic residues, which altogether exhibit a hydrophilic hydropathy profile. Like the deletional (truncated) heavy chain, these heavy chains are secreted into the culture medium of recombinant hosts.

Glycosylation variants include variants completely lacking in glycosylation (unglycosylated) and variants having at least one less glycosylated site than the native form (deglycosylated) as well as variants in which the glycosylation has been changed. Included are deglycosylated and unglycosylated amino acid sequence variants, deglycosylated and unglycosylated subunits having the native, modified amino acid sequence. For example, substitutional or deletional mutagenesis is employed to eliminate the N-or 0-linked glycosylation sites of the subunit, e.g., the asparagine residue is deleted or substituted for by another basic residue such as lysine or histidine. Alternatively, flanking residues making up the glycosylation site are substituted or deleted, even though the asparagine residues remain unchanged, in order to prevent glycosylation by eliminating the glycosylation recognition site. Additionally, unglycosylated MHC monomers which have the amino acid sequence of the native monomers are produced in recombinant prokaryotic cell culture because prokaryotes are incapable of introducing glycosylation into polypeptides.

Glycosylation variants are conveniently produced by selecting appropriate host-cells or by *in vitro* methods. Yeast, for example, introduce glycosylation which varies significantly from that of mammalian systems. Similarly, mammalian cells having a different species (e.g., hamster, murine, insect, porcine, bovine or ovine) or tissue origin (e.g., lung, liver, lymphoid, mesenchymal or epidermal) than the MHC source are routinely screened for the ability to introduce variant glycosylation as characterized for example by elevated levels of mannose or variant ratios of mannose, fucose, sialic acid, and other sugars typically found in mammalian glycoproteins. In vitro processing of the subunit typically is accomplished by enzymatic hydrolysis, e.g., neuraminidase digestion.

MHC glycoproteins suitable for use in the present invention have been isolated from a multiplicity of cells using a variety of techniques including solubilization by treatment with papain, by treatment with 3M KCl, and by treatment with detergent. For example, detergent extraction of Class I protein followed by affinity purification can be used. Detergent can then be removed by dialysis or selective binding beads. The molecules can be obtained by isolation from any MHC I bearing cell, for example from an individual suffering from a targeted cancer or viral disease.

Isolation of individual heavy chain from the isolated MHC glycoproteins is easily achieved using standard techniques known to those skilled in the art. For example, the heavy chain can be separated using SDS/PAGE and electroelution of the heavy chain from the gel (see, e.g., Dornmair et al., supra and Hunkapiller, et al., Methods in Enzymol. 91:227-236 (1983). Separate subunits from MHC I molecules are also isolated using SDS/PAGE followed by electroelution as described in Gorga et al. J. Biol. Chem. 262:16087-16094 (1987) and Dornmair et al. Cold Spring Harbor Symp. Quant. Biol. 54:409-416 (1989) Those of skill will recognize that a number of other standard methods of separating molecules can be used, such as ion exchange chromatography, size exclusion chromatography or affinity chromatography.

Alternatively, the amino acid sequences of a number of Class I proteins are known, and the genes have been cloned, therefore, the heavy chain monomers can be expressed using recombinant methods. These techniques allow a number of modifications of the MHC monomers as described above. For instance, recombinant techniques provide methods for carboxy terminal truncation which deletes the hydrophobic transmembrane domain. The carboxy termini can also be arbitrarily chosen to facilitate the conjugation of ligands or labels, for example, by introducing cysteine and/or lysine residues into the molecule. The synthetic gene will typically include restriction sites to aid insertion into expression vectors and manipulation of the gene sequence. The genes encoding the appropriate subunits are then inserted into expression vectors, expressed in an appropriate host, such as *E. coli,* yeast, insect, or other suitable cells, and the recombinant proteins are obtained.

As the availability of the gene permits ready manipulation of the sequence, a second generation of construction includes chimeric constructs. The α₁, α₂, α₃, domains of the class I heavy chain are linked typically by the α₃ domain of Class I with beta-2 microglobulin and coexpressed to stabilize the complex. The transmembrane and intracellular domains of the Class I gene can optionally also be included.

Construction of expression vectors and recombinant production from the appropriate DNA sequences are performed by methods known in the art. Standard techniques are used for DNA and RNA isolation, amplification, and cloning. Generally enzymatic reactions involving DNA ligase, DNA polymerase, restriction endonucleases, and the like, are performed according to the manufacturer's specifications. These techniques and various other techniques are generally performed according to Sambrook et al., Molecular Cloning--A Laboratory Manual, Cold Spring Harbor Laboratory, Cold Spring Harbor, N.Y., 1989. The procedures therein are believed to be well known in the art.

Expression can be in procaryotic or eucaryotic systems. Suitable eucaryotic systems include yeast, plant and insect systems, such as the *Drosophila* expression vectors under an inducible promoter. Procaryotes most frequently are represented by various strains of *E*. *coli.* However, other microbial strains may also be used, such as bacilli, for example *Bacillus subtilis,* various species of *Pseudomonas,* or other bacterial strains. In such procaryotic systems, plasmid vectors which contain replication sites and control sequences derived from a species compatible with the host are used. For example, *E. coli* is typically transformed using derivatives of pBR322, a plasmid derived from an *E. coli* species by Bolivar et al., Gene (1977) 2:95. Commonly used procaryotic control sequences, which are defined herein to include promoters for transcription initiation, optionally with an operator, along with ribosome binding site sequences, including such commonly used promoters as the β-lactamase (penicillinase) and lactose (lac) promoter systems (Change et al., Nature (1977) 198:1056) and the tryptophan (trp) promoter system (Goeddel et al., Nucleic Acids Res. (1980) 8:4057) and the lambda-derived P_{L} promoter and N-gene ribosome binding site (Shimatake et al., Nature (1981) 292:128). Any available promoter system compatible with procaryotes can be used.

The expression systems useful in the eucaryotic hosts comprise promoters derived from appropriate eucaryotic genes. A class of promoters useful in yeast, for example, include promoters for synthesis of glycolytic enzymes, including those for 3-phosphoglycerate kinase (Hitzeman, et al., J. Biol. Chem. (1980) 255:2073). Other promoters include, for example, those from the enolase gene (Holland, M. J., et al. J. Biol. Chem. (1981) 256:1385) or the Leu2 gene obtained from YEp13 (Broach, J., et al., Gene (1978) 8:121). A *Drosophila* expression system under an inducible promoter (Invitrogen, San Diego, CA) can also be used.

Suitable mammalian promoters include the early and late promoters from SV40 (Fiers, et al., Nature (1978) 273:113) or other viral promoters such as those derived from polyoma, adenovirus II, bovine papilloma virus or avian sarcoma viruses. Suitable viral and mammalian enhancers are cited above.

The expression system is constructed from the foregoing control elements operably linked to the MHC sequences using standard methods, employing standard ligation and restriction techniques which are well understood in the art. Isolated plasmids, DNA sequences, or synthesized oligonucleotides are cleaved, tailored, and religated in the form desired.

Site-specific DNA cleavage is performed by treatment with the suitable restriction enzyme (or enzymes) under conditions which are generally understood in the art, and the particulars of which are specified by the manufacturer of these commercially available restriction enzymes. In general, about 1 µg of plasmid or DNA sequence is cleaved by one unit of enzyme in about 20 µl of buffer solution; an excess of restriction enzyme may be used to insure complete digestion of the DNA substrate. After each incubation, the protein is removed by extraction with phenol/chloroform, and may be followed by ether extraction, and the nucleic acid recovered from aqueous fractions by precipitation with ethanol followed by running over a Sephadex G-50 spin column. If desired, size separation of the cleaved fragments may be performed.

Restriction cleaved fragments may be blunt ended by treating with the large fragment of E. coli DNA polymerase I (Klenow) in the presence of the four deoxynucleotide triphosphates (dNTPs). After treatment with Klenow, the mixture is extracted with phenol/chloroform and ethanol precipitated followed by running over a Sephadex G-50 spin column.

Synthetic oligonucleotides are prepared using commercially available automated oligonucleotide synthesizers. In the proteins of the invention, however, a synthetic gene is conveniently employed. The gene design can include restriction sites which permit easy manipulation of the gene to replace coding sequence portions with these encoding analogs.

Correct ligations for plasmid construction can be confirmed by first transforming E. coli strain MM294 obtained from E. coli Genetic Stock Center, CGSC #6135, or other suitable host, with the ligation mixture. Successful transformants can be selected by ampicillin, tetracycline or other antibiotic resistance or by using other markers depending on the mode of plasmid construction, as is understood in the art. Plasmid from the transformants are then prepared, optionally following chloramphenicol amplification. The isolated DNA is analyzed by restriction and/or sequenced by the dideoxy method of Sanger, F., et al., Proc. Natl. Acad. Sci. USA (1977) 74:5463 as further described by Messing, et al., Nucleic Acids Res. (1981) 9:309, or by the method of Maxam, et al., Methods in Enzymology (1980) 65:499.

The constructed vector is then transformed into a suitable host for production of the protein. Depending on the host-cell used, transformation is done using standard techniques appropriate to such cells. The calcium treatment employing calcium chloride, as described by Cohen, S. N., Proc. Natl. Acad. Sci. USA (1972) 69:2110, or the RbCl method described in Maniatis, et al., Molecular Cloning: A Laboratory Manual (1982) Cold Spring Harbor Press, p. 254 is used for procaryotes or other cells which contain substantial cell wall barriers. For mammalian cells without such cell walls, the calcium phosphate precipitation method of Graham and van der Eb, Virology (1978) 52:546 or electroporation is preferred. Transformations into yeast are carried out according to the method of Van Solingen, P., et al., J. Bacter. (1977) 130:946 and Hsiao, C. L., et al., Proc. Natl. Acad. Sci. USA (1979) 76:3829.

The transformed cells are then cultured under conditions favoring expression of the MHC sequence and the recombinantly produced protein recovered from the culture.

### MHC-binding Peptides

It is believed that the presentation of antigen by the MHC glycoprotein on the surface of antigen-presenting cells (APCs) occurs subsequent to the hydrolysis of antigenic proteins into smaller peptide units. The location of these smaller segments within the antigenic protein can be determined empirically. These MHC-binding peptides are thought to be about 8 to about 10, possibly about 8 to about 11, or about 8 to about 15 residues in length, and contain both the agretope (recognized by the MHC molecule) and the epitope (recognized by T-cell receptor on the T-cell). The epitope is a contiguous or noncontiguous sequence of about 5-6 amino acids that is recognized by the antigen-specific T-cell receptor..The agretope is a continuous or noncontiguous sequence that is responsible for binding of the peptide with the MHC glycoproteins. The invention provides kits, and assays for evaluating putative MHC-binding peptides, or a library of such peptides, to determine whether such fragments can activate a peptide-specific T-cell, for example when contained within a mixed population of T-cells, when incorporated into a ternary complex with an MHC monomer or modified MHC monomer. Optionally, the MHC monomers can be incorporated into MHC multimers, for example, MHC tetramers, for use in the invention assays and kits.

Thus, the invention provides screening methods to be used in screening of candidate peptides for use in diagnostic assays, vaccines, and other treatment modalities. Putative MHC-binding peptides for use in the invention methods can be made using any method known in the art, the most convenient being peptide synthesis for fragments of 8 to 15 amino acids in length.

As used herein to describe the invention assays, the terms "peptide exchange" and "exchanged peptide" refer to a competition assay wherein three peptides compete in solution for binding to the binding pocket of an MHC monomer or modified MHC monomer. At the start of the competition assay, the three peptides are: (1) an unlabelled MHC-binding peptide, referred to as a "template peptide," which is specific for and is initially bound in the binding pocket of the monomer; (2) an unlabelled, initially unbound test or putative MHC-binding peptide of unknown affinity and/or unknown specificity, referred to as a "competitor peptide"; and (3) a detectably labeled, initially unbound "tracer peptide" that is specific for and has a affinity for the binding pocket that is higher than that of the "template peptide" The template peptide is selected to have low or medium affinity for the binding pocket so that it is readily is dissociated from the MHC ternary complex and replaced in solution either by a competitor peptide or a tracer peptide. Successful competition of the competitor peptide for the binding pocket indicates the competitor peptide has higher affinity for the binding pocket than either the template peptide or the detectably labeled tracer peptide. Similarly, successful competition of the tracer peptide for the binding pocket indicates the tracer peptide has higher affinity for the binding pocket than both the template peptide and the detectably labeled tracer peptide. Thus, the tracer peptide and template peptide are selected to establish a minimum affinity for any competitor peptide that is successful in the invention competition assay since a competitor peptide becomes an "exchanged" peptide only if the affinity of the competitor peptide is sufficient under the assay conditions to compete successfully for binding to the binding pocket. A monomer in which the template peptide has been replaced (i.e. exchanged by a higher affinity competitor peptide) is referred to for convenience as an "exchanged monomer." A monomer in which the template peptide has been replaced (i.e. exchanged) by a tracer peptide is referred to herein for convenience as a "tracer monomer." Two peptides that bind to an MHC Class I molecule can be exchanged in solution using this procedure as readily as on a cell surface without the binding peptide being folded into the binding pocket during formation of a ternary complex. The procedures and definitions of low and medium affinity used in peptide exchange are described in full in U.S. Patent application Serial No. 10/782, 664, filed February 18,2004.

In addition to their respective affinities, concentration of the competitor peptide and tracer peptide in the assay solution is also an important consideration in establishing the liquid assay conditions. The competitor peptide is provided in molar excess to allow for optimum binding opportunity, with about 100-fold molar excess being the preferred amount of excess.

The MHC monomer used in the invention systems and methods can be any MHC monomer or modified MHC monomer, e.g., comprising a class I heavy chain that binds a peptide in the range of 8 to 11 amino acids, for example 8 to 15 amino acids and beta-2 microglobulin under renaturing conditions. The MHC monomer heavy chain can be encoded by any partial or full-length modified or unmodified MHC gene sequence from any species or subtype, or a combination thereof, including without limitation human and murine species, and chimera thereof. Preferred MHC encoding gene sequences are those encoding any HLA allele genotype and any variation or polymorphism thereof. For example, the MHC monomer utilized in the invention systems and methods can comprise any partial or full- length HLA heavy chain that binds an HLA-binding peptide and beta-2 microglobulin under renaturing conditions, i.e., any subtype or allele of HLA-A, HLA-B, or HLA-C.

For example, in one embodiment, the MHC heavy chain in the monomer is modified by truncation to include only the al, a2 and the a3 domains of an HLA heavy chain. In still another embodiment, the heavy chain in the MHC monomer can be a chimeric, such as a fusion protein, containing these MHC domains and an anchor domain, wherein the MHC domain binds to a MHC-binding a peptide, as described herein, while the anchor domain is suitable for immobilizing the MHC monomer to a surface. The anchor domain can be a polypeptide fused with the HLA domain to form a fusion protein or can be any entity coupled to the HLA domain through any suitable means known in the art, e.g., biotinylated MHC monomer.

The MHC monomer can be attached to the solid surface by any suitable means known in the art. For example, the MHC monomer can be immobilized to a surface either directly or indirectly, e.g., via an anchoring or connecting entity. In one embodiment, the solid surface of the invention system is coated with a first ligand entity, which binds to or interacts with a second ligand connected to or within the MHC monomer, e.g., via covalent or noncovalent bond. In another embodiment, the surface is coated with avidin or its derivatives, e.g., streptavidin, and the MHC monomer contains biotin or its derivatives as its anchor domain. Attachment of the MHC monomer to the solid surface, in one embodiment of the invention, is reversible or cleavable.

In an alternative procedure by which peptides contained in MHC monomers coated or immobilized to a solid surface can be switched, the monomers are denatured, e. g., stripped or dissociated in a denaturing condition, and then renatured, e. g., refolded from a denatured form under renaturing conditions so as to bind an desired MHC-binding peptide. The solid surface used in the invention methods is preferably one belonging to an invention system or kit and is prepared as described herein. If the MHC monomers attached to the solid surface at the start of the assay procedure are in a reconstituted form, test peptides can be inserted into the MHC monomers for the assay by exposure to denaturing conditions as described herein, for example by exposure to a pH in the range from about 2 to about 4, or exposure overnight to a temperature higher than about 37 C. After denaturation, unbound MHC-binding peptides are washed away.

The term"MHC multimer"or"multimeric MHC monomer or modified MHC monomer complex"is used herein to refer to a complex containing two or more MHC monomers, usually bound together via a multivalent entity. An MHC multimer can comprise an MHC dimer, MHC trimer, MHC tetramer, and the like (see, for example, U. S. Pat. No. 5,635, 363). The MHC monomers in an MHC multimer can also be linked directly, for example, through a disulfide bond, or indirectly, for example, through a specific binding pair, and also can be associated through a specific interaction between secondary or tertiary structures of the monomers, such as a leucine zipper, which can be engineered, for example, into a MHC class I molecule component of the monomers. MHC tetramers are complexes of four MHC monomers, which are associated with a specific peptide antigen and contain a fluorochrome.

The monomers of an MHC tetramer or other MHC multimer can be operatively linked together, covalently or non-covalently, and directly through a physical association or chemical bond or indirectly through the use of a specific binding pair or by attachment to a multivalent entity through the use of a specific binding pair. Alternatively, the monomers of an MHC multimer can be operatively linked to a multivalent entity containing multiple specific attachment sites for MHC monomers. As used herein, the term "operatively linked" or "operatively associated" means that a first molecule and at least a second molecule are joined together, covalently or non-covalently, such that each molecule substantially maintains its original or natural function. For example, where two or more MHC monomers, each of which can specifically bind a peptide antigen, are operatively linked to form an MHC multimer, each of the two or more MHC monomers in the MHC multimer maintains its ability to specifically bind the peptide antigen. Any means can be used for operatively linking the monomers, provided it does not substantially reduce or inhibit the ability of an MHC multimer to present an antigenic peptide to a T-cell. Generally, the MHC monomers are linked together or to a multimeric moiety through the heavy chain component of the monomers. Thus, the monomers can be linked, for example, through an interchain peptide bond formed between reactive side groups of the amino acids comprising the heavy chains, through interchain disulfide bonds formed between cysteine residues in the heavy chains, or through any other type of bond that can generally be formed between the chemical groups represented by the amino acid side chains. A convenient means for operatively linking the monomers of an MHC multimer to a multivalent entity utilizes specific attachment sites that are each part of a binding pair. Where the MHC multimer is formed by attachment of the MHC monomers to a multimeric moiety, the monomers and the multivalent entity each provide one of the specific attachment sites that make up a binding pair.

For example, the heavy chains of the monomers can be biotinylated and four monomers can be formed into tetramers by chemical coupling to streptavidin, which naturally has 4 biotin-binding sites.

The term "antibody" is used broadly herein to include polyclonal and monoclonal antibodies, as well as antigen binding fragments of such antibodies, such as a Fab. Depending on the particular method of the invention, antibodies having an Fc region can be useful or various specificities can be useful, including an antibody, or antigen-binding fragment thereof, that specifically binds a peptide ligand on a cell surface, such as a tumor marker.

The term "specifically binds" or "specific for," when used in reference to an antibody means that an interaction of the antibody and a particular epitope has a dissociation constant of at least about 1 x 10⁻⁶, generally at least about 1 x 10⁻⁷, usually at least about 1 x 10⁻⁸, and particularly at least about 1 x 10⁻⁹ or 1 x 10⁻¹⁰ or less. As such, Fab, F(ab')₂, Fd and Fv fragments of an antibody that retain specific binding activity for a β2-microglobulin epitope are included within the definition of an antibody. The term "specifically binds" or "specifically interacts" is used similarly herein to refer to the interaction of members of a specific binding pair, as well as to an interaction between β2-microglobulin and an MHC class I heavy chain.

The term "antibody" as used herein includes naturally occurring antibodies as well as non-naturally occurring antibodies, including, for example, single chain antibodies, chimeric antibodies, bifunctional antibodies and humanized antibodies, as well as antigen-binding fragments thereof. Such non-naturally occurring antibodies can be constructed using solid phase peptide synthesis, can be produced recombinantly or can be obtained, for example, by screening combinatorial libraries consisting of variable heavy chains and variable light chains (see Huse et al., Science 246:1275-1281, 1989). These and other methods of making, for example, chimeric, humanized, CDR-grafted, single chain, and bifunctional antibodies are well known to those skilled in the art (Winter and Harris, Immunol. Today 14:243-246, 1993; Ward et al., Nature 341:544-546, 1989; Harlow and Lane, Antibodies: A laboratory manual (Cold Spring Harbor Laboratory Press, 1988); Hilyard et al., Protein Engineering: A practical approach (IRL Press 1992); Borrabeck, Antibody Engineering, 2d ed. (Oxford University Press 1995)).

An antibody having a desired specificity can be obtained using well-known methods. For example, an antibody having substantially the same specific binding activity of C21.48A can be prepared using methods as described by Liabeufet al. (*supra,* 1981) or otherwise known in the art (Harlow and Lane, Antibodies: A laboratory manual (Cold Spring Harbor Laboratory Press 1988)). For example, an antibody that specifically binds a specific peptide ligand on the surface of a cell can be obtained using the tumor marker Fc receptor or a peptide portion thereof as an immunogen and removing antibodies that bind with other antigens. A peptide portion of a cell surface marker, one that is suitable for distinguishing a particular type of cell associated with a specific disease state, is present, for example, in a spatial region of the marker that is exposed on the cell surface when expressed by the cell. and can be identified using crystallographic data or well known protein modeling methods (see, for example, Shields et al., J. Immunol. 160: 2297-2307,1998 ; Pedersen et al., Eur, J. Immunol. 25: 1609,1995 ; Evans et al., Proc. Natl. Acad. Sci., USA 79: 1994,1995 ; Garboczi et al., Proc. Natl. Acad. Sci., USA 89: 3429-3433, 1992.

Monoclonal antibodies also can be obtained using methods that are well known and routine in the art (Kohler and Milstein, Nature 256 : 495,1975 ; Coligan et al., *supra,* 1992, sections 2.5. 1-2.6. 7 ; Harlow and Lane, *supra,* 1988). For example, spleen cells from a mouse immunized with a tumor marker or peptide tag, or an epitopic fragment thereof, can be fused to an appropriate myeloma cell line such as SP/02 myeloma cells to produce hybridoma cells. Cloned hybridoma cell lines can be screened using, for example, labeled β2-microglobulin to identify clones that secrete monoclonal antibodies having the appropriate specificity, and hybridomas expressing antibodies having a desirable specificity and affinity can be isolated and utilized as a continuous source of the antibodies. Polyclonal antibodies similarly can be isolated, for example, from serum of an immunized animal. Such isolated antibodies can be further screened for the inability to specifically bind the cell surface tumor marker an Fc receptor or an expressed peptide tag on a cell surface. Such antibodies, in addition to being useful for performing a method of the invention, also are useful, for example, for preparing standardized kits.

Monoclonal antibodies, for example, can be isolated and purified from hybridoma cultures by a variety of well-established techniques, including, for example, affinity chromatography with Protein-A SEPHAROSE gel, size exclusion chromatography, and ion exchange chromatography (Bames et al., in Meth. Mol. Biol. 10: 79-104 (Humana Press 1992); Coligan et al., *supra,* 1992, see sections 2.7. 1-2.7. 12 and sections 2.9. 1-2.9. 3). Methods of *in vitro* and *in vivo* multiplication of monoclonal antibodies are well known. For example, multiplication *in vitro* can be carried out in suitable culture media such as Dulbecco's Modified Eagle Medium or RPMI 1640 medium, optionally replenished by a mammalian serum such as fetal calf serum or trace elements and growth sustaining supplements such as normal mouse peritoneal exudate cells, spleen cells, bone marrow macrophages. Production *in vitro* provides relatively pure antibody preparations and allows scale-up to yield large amounts of the desired antibodies. Large-scale hybridoma cultivation can be carried out by homogenous suspension culture in an airlift reactor, in a continuous stirrer reactor, or in immobilized or entrapped cell culture. Multiplication *in vivo* can be carried out by injecting cell clones into mammals histocompatible with the parenT-cells, for example, syngeneic mice, to cause growth of antibody-producing tumors. Optionally, the animals can be primed with a hydrocarbon, for example, oil such as pristane (tetramethylpentadecane) prior to injection. After one to three weeks, the desired monoclonal antibody is recovered from the body fluid of the animal.

Antibodies and antigen binding fragments of antibodies useful in practice of the invention methods for determining activation of T-cells. An antigen-binding fragment of an antibody can be prepared by proteolytic hydrolysis of a particular antibody such as C21.48A, or by expression in *E. coli* of DNA encoding the fragment. Antibody fragments can be obtained by pepsin or papain digestion of whole antibodies by conventional methods. For example, antibody fragments can be produced by enzymatic cleavage of antibodies with pepsin to provide a 5S fragment denoted F(ab')₂. This fragment can be further cleaved using a thiol reducing agent, and optionally a blocking group for the sulfhydryl groups resulting from cleavage of disulfide linkages, to produce 3.5S Fab' monovalent fragments. Alternatively, an enzymatic cleavage using pepsin produces two monovalent Fab' fragments and an Fc fragment directly (see, for example, Goldenberg, U.S. Patent No. 4,036,945 and U.S. Pat. No. 4,331,647; Nisonhoff et al., Arch. Biochem. Biophys. 89:230. 1960; Porter, Biochem. J. 73:119, 1959; Edelman et al., Meth. Enzymol., 1:422 (Academic Press 1967); Coligan et al., *supra,* 1992, see sections 2.8.1-2.8.10 and 2.10.1-2.10.4).

Methods of cleaving antibodies, such as separation of heavy chains to form monovalent light/heavy chain fragments, further cleavage of fragments, or other enzymatic, chemical, or genetic techniques can also be used, provided the fragments specifically bind to the antigen that is recognized by the intact antibody. For example, Fv fragments comprise an association of variable heavy (V_{H}) chains and variable light (V_{L}) chains, which can be a noncovalent association (Inbar et al., Proc. Natl. Acad. Sci., USA 69:2659, 1972). Alternatively, the variable chains can be linked by an intermolecular disulfide bond or crosslinked by chemicals such as glutaraldehyde (Sandhu, Crit. Rev. Biotechnol. 12:437, 1992).

Although not a necessity for *in vitro* uses, humanized monoclonal antibodies also can be used in formation of a bridging complex, a method or kit of the invention if desired. Humanized monoclonal antibodies can be produced, for example, by transferring nucleotide sequences encoding mouse complementarity determining regions from heavy and light variable chains of the mouse immunoglobulin into a human variable domain, and then substituting human residues in the framework regions of the murine counterparts. Methods for cloning murine immunoglobulin variable domains are known (see, for example, Orlandi et al., Proc. Natl. Acad. Sci., USA 86:3833, 1989), and for producing humanized Monoclonal antibodies are well known (see, for example, Jones et al., Nature 321:522, 1986; Riechmann et al., Nature 332:323, 1988; Verhoeyen et al., Science 239:1534, 1988; Carter et al., Proc. Natl. Acad. Sci., USA 89:4285, 1992; Singer et al., J. Immunol. 150:2844, 1993; Sandhu, *supra,* 1992).

Antibodies useful in a method of the invention also can be derived from human antibody fragments, which can be isolated, for example, from a combinatorial immunoglobulin library (see, for example, Barbas et al., Methods: A Companion to Methods in Immunology 2:119, 1991; Winter et al., Ann. Rev. Immunol. 12:433, 1994). Cloning and expression vectors that are useful for producing a human immunoglobulin phage library are commercially available (Stratagene; La Jolla CA). In addition, the antibody can be derived from a human monoclonal antibody, which can be obtained from transgenic mice that have been "engineered" to produce specific human antibodies in response to antigenic challenge (see, for example, by Green et al., Nature Genet. 7:13, 1994; Lonberg et al., Nature 368:856, 1994; and Taylor et al., Int. Immunol. 6:579, 1994; see, also, Abgenix, Inc.; Fremont CA).

Accordingly, in one embodiment the invention provides a method of identifying a peptide capable of activating a peptide-specific T-cell, said method comprising: a) incubating under suitable conditions peptide-specific T-cells and a solid surface having attached thereto: 1) BSA-biotin-avidin coated onto the solid surface; 2) a plurality of biotinylated MHC monomers or modified biotinylated MHC monomers having a bound candidate MHC-binding peptide wherein the biotinylated MHC monomers or modified biotinylated MHC monomers are bound via the biotin to the avidin; and 3) a biotinylated anti-TNF alpha or anti-IFN gamma antibody that forms an antibody-TNF alpha or -IFN gamma complex upon activation of the peptide-specific T-cells by the biotinylated MHC monomer - MHC-binding peptide complex; and b) contacting the antibody-TNF alpha or IFN gamma complex obtained from a) with a second antibody specific for TNF alpha or IFN gamma and wherein the second antibody is tagged with a detectable label so as to allow formation of an antibody-TNF alpha or -IFN gamma-antibody complex; and c) detecting a signal produced by the detectable label, wherein the detecting indicates activation of the peptide-specific T-cells and wherein the activation of the peptide-specific T cells indicates that the bound candidate MHC-binding peptide is capable of activating the peptide-specific T-cells. Preferably, the first and second antibodies bind to different epitopes of the selected soluble T-cell activation factor and are monoclonal antibodies.

In preparation for the invention assay, an avidin-coated 96-well microtiter plates are coated first with the desired MHC Class I or II allele monomers containing bound peptides of interest. Then, the same plates are coated secondarily with a biotinylated antibody recognizing a soluble T-cell activation factor to be analyzed and then the plates are washed and T-cell sample to be tested is added. The plates are incubated over night, for example for 12 hours or up to about 24 hours to allow activation of specific T-cells in the sample by the peptide in the monomer. The T-cell lines tested for activation can be polyclonal and the specificity of the peptide can be known or the T-cells can be of a known specificity and the assay can be used to determine whether one or more peptides, for example, out of a group of peptides of unknown specificity will activate the T-cells.

After cell activation the plates are washed to remove unbound cells and a detectably labeled second antibody directed to the same soluble factor (for example, directly conjugated to an enzyme or a fluorophore and recognizing a different epitope of the soluble factor) is added. The enzymatic activity is revealed using known methods and the signal obtained is proportional to the degree of T-cell activation (e. g. , the number of activated T-cells) in the sample.

The T-cells assayed for activation can be contained in a sample comprising a mixed population of T-cells, such PBMCs or whole blood from a patient sample or from a donor. The invention method can further comprise enriching the mixture of T-cells for MHC class I monomers prior to the incubation, for example by using an anti-CD8 antibody to identify CD8⁺ cells in the mixture or for MHC class II monomers by using an anti CD4 antibody to identify CD4⁺ cells.

When a mixed population of T-cells is assayed according to the invention methods, and the MHC monomers are designed to contain the same test or putative MHC-binding peptide, the magnitude of the signal determined is proportional to the number of peptide- activated T-cells contained in the sample. Thus, the assay can be repeated using. different known or putative MHC-binding peptides and the results compared to screen peptides to determine the relative effectiveness of the peptides for activating a T-cells having a known antigen-specificity. For example, a library of peptides can be screened to determine their relative antigen-specificity for an antigen to be associated with a particular disease, such as a cancer or a viral infection.

However, as is known in the art, certain peptides can cause cross-activation of two or more populations of T-cells contained in a mixed-population sample. Then the specificity of the peptide needs to be confirmed by conducting an additional assay, such as a MHC tetramer T-cell"staining"assay as is known in the art and described in U.S. Patent No. 5,635, 363, to determine that the peptide activates T-cells in an antigen-specific manner.

On the other hand, when the population of T-cells assayed is homogeneous and a known number of cells are employed in the assay, the magnitude of the signal obtained from the complete population of T-cells can be used to assay the effectiveness of a particular MHC-binding peptide for activating the T-cells. As in other embodiments of the invention methods, whether the T-cell activation is also antigen-specific can be determined by conducting an additional assay, such as a MHC tetramer T-cell "staining" assay to determine that T-cells identified as activated are activated in an antigen-specific manner.

Suitable conditions for allowing T-cell activation to occur according to the invention methods can include a temperature in the range from about 18°C to about 37°C, for example room temperature, and an incubation period of from about 3 to about 48 hours, for example about 8 hours or overnight. The suitable conditions can further include the absence of sodium azide.

A soluble T-cell activation factor, as the term is used herein, is any soluble factor produced by an activated T-cell and not by an unactivated T-cell and for which a suitable antibody is known or can be produced using the methods known in the art and as described herein. For example, the soluble T-cell activation factor can be a cytokine, such as interferon gamma or tumor necrosis factor; a chemokines, such as MIP-1 alpha or RANTES; lytic or apoptotic factor, such as perforin, granzyme or FasL. For example, interferon gamma and TNF alpha antibodies are available from Immunotech (Marseilles, France). Additional antibodies are commercially available from vendors such as R&D systems, SEROTEC, Diaclone, and the like. If the soluble factor selected for the assay is one associated with a disease state, and the T-cells are from an identified donor, the invention assay methods can be used to detect or monitor a patient-donor's immune response to the disease state.

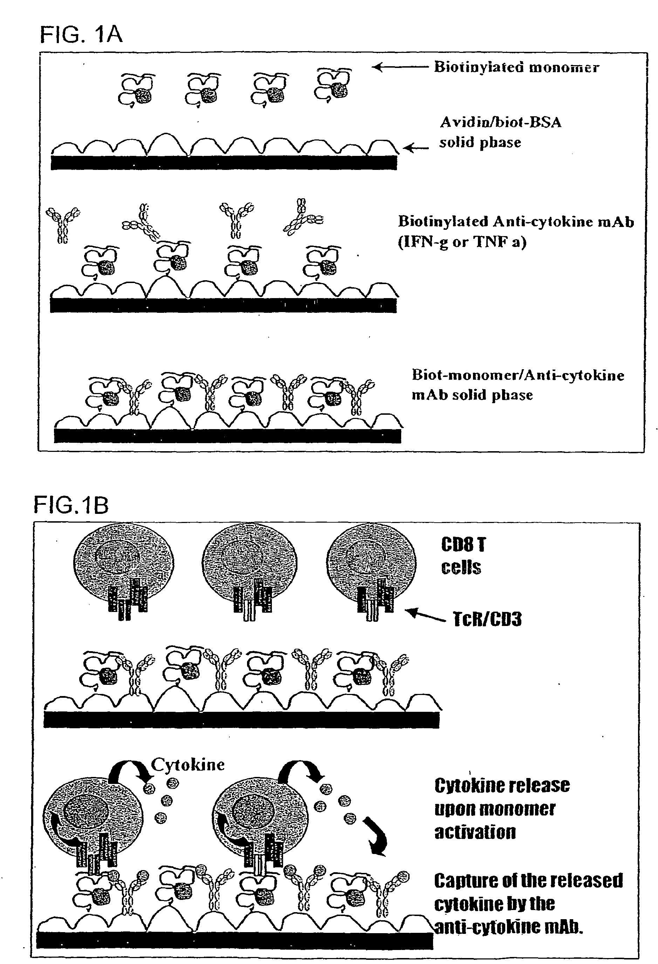

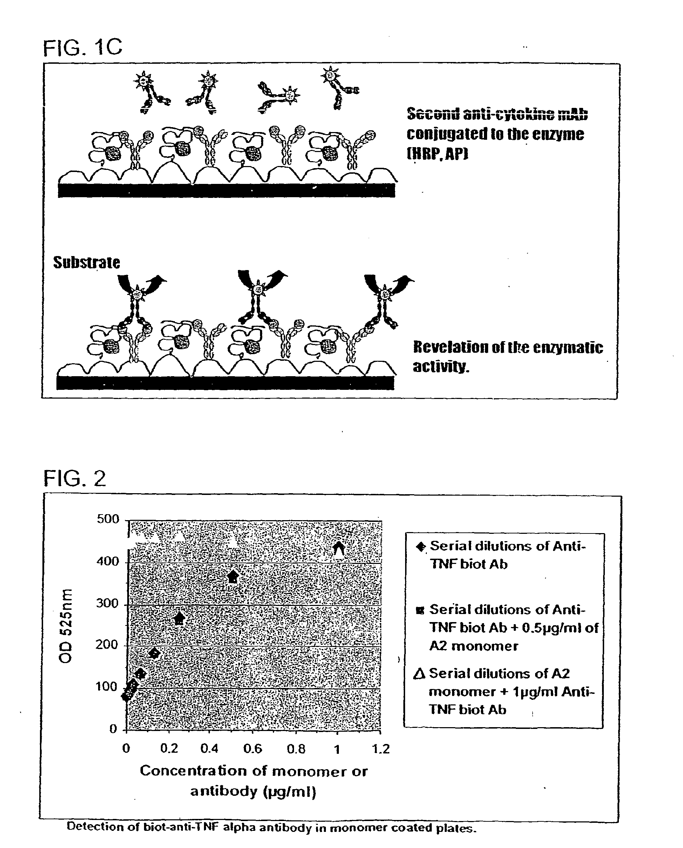

Schematic representations of the invention soluble T-cell activation factor assay appear in Figs. 1A-C and following:

| Step 1 | Step 2 | Step 3 | Step 4 | Step 5 |
|---|---|---|---|---|
| Incubation of biotinylated-monomer in avidin coated plates. | Washing, incubation with biotinylated-anti-soluble factor antibody | Washing and incubation of T-cell sample overnight | Washing and incubation with the second labeled antibody | Washing and detection |

In another embodiment, the invention provides methods for determining activation of a peptide-specific T-cell by a MHC-binding peptide bound to an MHC monomer or modified MHC monomer. In this embodiment of the invention, peptide-specific T-cells and a solid surface having attached thereto a plurality of MHC monomers or modified MHC monomers, each having bound a putative MHC-binding peptide for which specificity of the T-cells is unknown, and an antibody specific for a selected surface T-cell activation marker are incubated r under suitable conditions. The suitable conditions are selected to allow formation of an antibody-surface activation marker complex on the surface of a peptide-specific T-cell that is activated by the putative MHC-binding peptide so as to produce the T-cell activation marker. Activation of the T-cells by the MHC-binding peptide is indicated by detecting formation of the antibody-surface activation marker complex on T-cells obtained from the incubation. The antibody is preferably, but not always, a monoclonal antibody. Any method of detecting formation of the antibody-surface marker complex known in the art can be used. Preferably antibody is tagged with a detectable label and, after washing away unbound components from the cell surface, formation of the complex can be determined by detecting the presence on the cell surface of the detectably labeled antibody.

For example, the detectable label used to tag the antibody that binds specifically to the T-cell activation marker can be fluorescent, such as FITC or PE, and the detecting can comprise utilizing flow cytometry to indicate the number of T-cells in the sample that are tagged with the antibody. Although any T-cell activation marker can be selected for which a specific antibody is known, it is presently preferred to utilize CD25 or CD69 as the surface activation marker. Other surface activation markers, such as HLA-DR, CD134, CD2R,CD26, CD49b, CD54, CTLA-4, and CD100 may also be used as the activation marker. Monoclonal antibodies specific for CD25, such as 1HT44H3, B1.49.9, 33B3.1 and anti-CD69 mAbs TP1.55.3 are available from Immunotech (Marseilles) Anti-CD25 Clone M-A251 and anti-CD69 Clone FN50 are commercially available from BD Biosciences (San Jose, CA).

In still another embodiment; the invention provides methods for determining the presence in whole blood or a peripheral blood mononucleocyte cell (PBMC)-containing sample of T-cells that are specifically activated by an epitope contained in an MHC-binding peptide. In this embodiment, the PBMCs are incubated under suitable conditions with MHC multimers having a bound MHC binding peptide and a first detectable label, so as to allow internalization of the MHC multimers by T-cells in the PBMCs that bind to the multimers. T-cells in the PBMCs that emit a signal produced by the first detectable label are detected, thereby identifying T-cells that specifically bind to an epitope in the MHC- binding peptide. Then the T-cells that specifically bind the MHC-binding peptide are further incubated with peptides in solution or monomers on solid phase before staining with an antibody tagged with a second detectable label, wherein the antibody is specific for a T-cell activation marker so as to allow formation of an antibody-activation marker complex. T-cells emitting the signals produced by both the first and the second detectable labels are identified as being specifically activated by an antigen contained in the MHC-binding peptide.

Alternatively, if the T-cells identified as emitting a signal from the first label are subjected to separate incubation with the antibody tagged with the second detectable label, T- cells emitting a signal produced by the second detectable label can be identified as being specifically activated by an antigen contained in the MHC-binding peptide.

In yet another embodiment, the invention provides kits useful in practice of the invention methods a kit comprising: a solid surface, wherein the surface has attached thereto: a) BSA-biotin-avidin; b) one or more biotinylated MHC monomers, modified biotinylated MHC monomers or biotinylated multimers thereof wherein the monomers contain a suitable MHC-binding peptide and wherein the biotinylated MHC monomers, modified biotinylated MHC monomers or biotinylated multimers thereof are bound via the biotin to the avidin; and c) a biotinylated anti-TNF alpha or anti-IFN gamma antibody. The invention kits comprise a solid surface, wherein the surface has attached thereto. The solid surface is preferably a bead or a microtiter plate with monomers or multimers attached to wells of the plate. The kit may further contain an instruction for use of the kits in the invention methods and a control peptide to which the MHC monomer binds in a reconstituted form.

The invention kits can be used as a cytokine detection kit for larger use, such as sample testing for known-epitopes, immune-monitoring, and the like, as described herein. Thus, the invention methods and kits can be used to diagnose a disease state or monitor the immune response to a disease state in a patient wherein the MHC binding peptide contains an antigen associated with a particular disease state and detection of the signal produced by the second detectable label indicates, respectively the donor-patient is producing the antigen associated with the particular disease and the donor-patient is producing T-cells that, when activated, can destroy cells that produce the antigen, for example as a surface cell marker.

The invention methods can be employed for all HLA class I and II molecules and can be combined with different monoclonal antibodies specific for detecting various secreted soluble T-cell activation factors, such as the cytokines particular to HLA Class I and II - restricted T-cells. This invention can be used to define HLA-restricted epitopes.

As the results of the invention methods have been shown herein to be proportional either to the number of T-cells tested or to the number of multimers used in the test, measured quantitatively, the amount of signal produced by a sample containing a known number of cells from the patient and/or a known number of multimers is an indicator of the proportion of the patient's T-cells that are antigen restricted, thus also indicating, at least in comparison with the proportion found in healthy individuals, the strength of the patient's immune response to the disease-associated condition. For monitoring severity of the disease-associated condition in the patient or the effects of treatment of the patient, the invention assays can be repeated at spaced intervals to monitor the patient's production of antigen-specific T-cells.

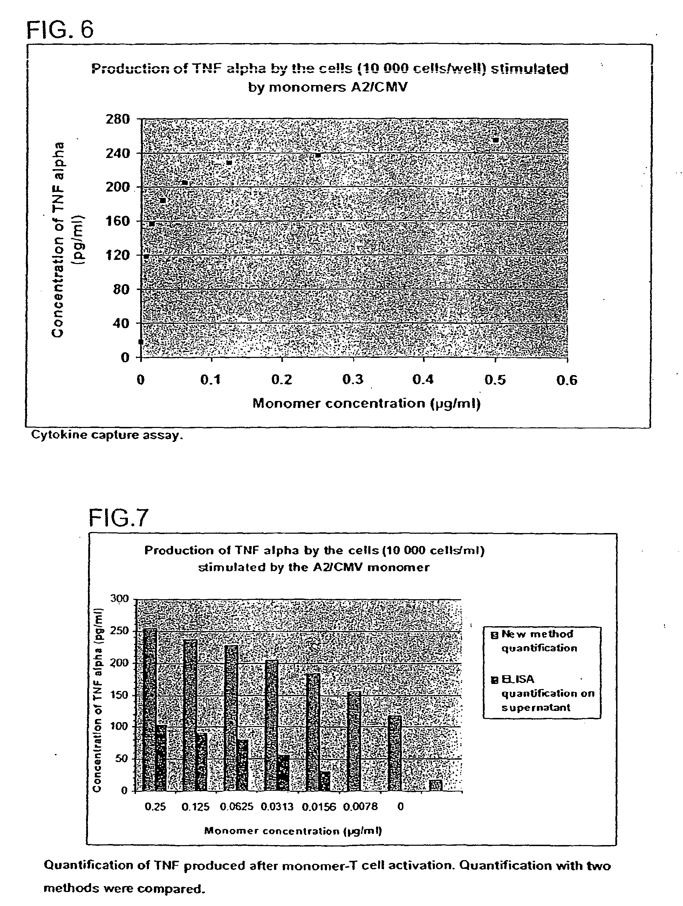

As shown by comparative results in Fig. 7, it has been discovered that the invention methods employing the dual antibody technique for quantification of soluble T-cell activation factor production by activated T-cells is more sensitive and reliable than conventional ELISA methods.

For the assay, the solid surface with attached denatured MHC monomers or modified MHC monomers can be incubated with a test or putative MHC-binding peptide, for example a member of a library of peptides, under reconstituting conditions for a suitable period of time to allow for formation of ternary complexes. The reconstituting conditions will include the presence of a sufficient amount of beta-2 microglobulin (or beta 2 microglobulin modified to increase binding or stabilize ternary complex formation) to saturate the MHC monomers. For example, it is contemplated that the beta 2-microglobulin may be modified by attachment thereto of a stabilizing molecule, such as a lucine zipper, or the like, to stabilize ternary complex formation. Incubation with the putative MHC-binding peptide and beta-2 microglobulin will typically be require from about 12 hours or overnight to about 48 hours to allow for complex formation. The reconstituting conditions may also include a temperature in the range from about 18 C to about 37 °C, for example about 4 °C to about 8 °C.

The solid surface coated with the MHC monomer used in the invention methods and kits can be dried and stored for use at a later time.

In one embodiment, the antibody used in the invention methods is provided with a detectable label, i.e., a label that produces a detectable signal as is known in the art. Labels may be conjugated to the antibody using any of a variety of procedures known in the art. Alternatively, the antibody can be produced to include a label, such as a radioactive amino acid. Labels suitable for use in the invention kits and methods include, but are not limited to, radioisotopes, fluorochromes, enzymes, biotin and electron dense molecules. Currently, enzymes for which contact with a substrate results in a color change are preferred for measurement of soluble factors. It is preferred that the coloring substrate have its absorption maximum wavelength in a visible region, be highly sensitive and readily distinguishable, and possess high stability over time. To meet such requirements, coloring substrates such as alkaline phosphatase enzyme are presently preferred. Such enzymatic labels can also be incorporated into the invention kits.

In embodiments of the invention wherein a T-cell surface activation marker is detected, a detection antibody specific for the T-cell surface activation marker, preferably a monoclonal antibody, can be used. Detection of signal produced by binding of the detection antibody to the T-cell surface activation markers (i.e., on T-cells) indicates those T-cells that have been activated. The result can be easily be detected and/or quantified after washing away unbound antibody and other components of the system by determining the signal intensity. The detectable label presently preferred for attachment to the antibody is a fluorescent label, e.g., FITC, or PE. The binding of fluorescently labeled antibodies to T-cells on the solid surface can readily be detected using a fluorimeter, by fluorescence determined flow cytometry; or any of the methods of high throughput fluorescence scanning known in the art.

The following examples are intended to illustrate but not to limit the invention in any manner, shape, or form, either explicitly or implicitly. While they are typical of those that might be used, other procedures, methodologies, or techniques known to those skilled in the art may alternatively be used.

### EXAMPLE 1

### Coating and Detection of Biotinylated monomer and biotinylated Anti-TNF alpha antibody.

In this experiment avidinated 96-well microtiter plates were coated with biotinylated class I HLA-A*0201 monomers and with anti-TNF alpha antibodies as follows. The concentration of the anti-TNF alpha antibody that can be bound by the monomer coated avidin plates was first determined. BSA-Biotin-Avidin coated 96-well plates for fluorimetric assay were used. For coating, HLA-A*0201 /CMVpp65 N9V monomer was incubated with the avidinated wells at 0.5 ug/ml. After coating, the plates were washed and incubated with different concentrations of biotinylated-anti-TNF alpha mAb (0, 0.0078, 0.0156, 0.03125, 0.0625, 0.125, 0.25, 0.5 and 1 ug/ml) to additionally coat the plates with the capture antibody.

After washing off unbound components, the concentration of the anti-TNF alpha antibody that can be bound by the monomer-coated plates was determined by adding 1 ug/ml of conjugated goat anti-mouse FITC antibody as the detector antibody. The detector antibody was incubated with the coated plates for 2 hours at room temperature under shaking. Plates were washed three times, and the bound fluorescence was read out. As a control, plates coated only with the anti-TNF alpha antibody, but without monomer, were used. As shown in Fig. 2, equivalent signals were found when the biotinylated anti-TNF alpha antibody was coated in the plates without monomer compared to the signal obtained in plates coated previously with the biotinylated-monomer.

To detect proper folding of the monomers on the plates, an anti-HLA-ABC antibody that binds to a conformational epitope present in a ternary complex but not present in an improperly assembled monomer complexes was incubated with the plates. The results showed that binding of the anti-HLA-ABC antibody was not impaired by the presence of the cytokine specific antibody co-coated with monomers. This shows that plates can be coated with both monomer and mAb in preparation for the assay.

As shown in Fig. 2, white open triangles (Δ) correspond to the data obtained when the monomer was diluted and the biotin-anti-TNF alpha antibody remained constant. After incubation with the goat anti-mouse antibody, the fluorescence was measured. The number of biotin binding sites used by the biotinylated antibody remains constant independently of the concentration of the monomer, meaning that there is no limitation to binding the needed amounts of antibody to the pre-coated plates.

### EXAMPLE 2

### TNF alpha ELISA

A second set of experiments was conducted to illustrate that the biotin-anti-TNF alpha antibody is able to capture TNF alpha in presence of the monomer at different concentrations, and this complex can be detected by a second monoclonal antibody directly conjugated to the alkaline phosphatase enzyme.

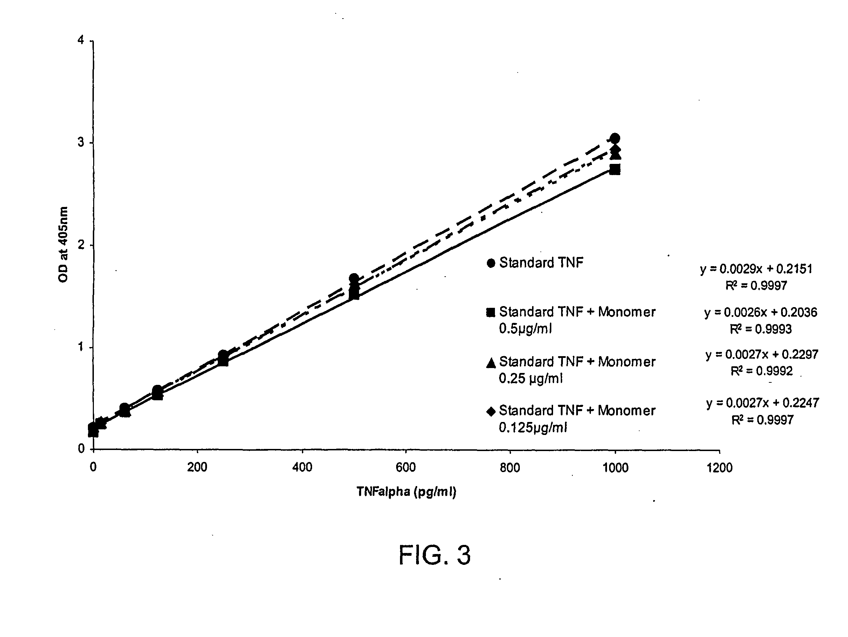

Different solutions of Monomer HLA-A*0201 CMVpp65 N9V at 0.5, 0.25 and 0.125 ug/ml were prepared and coated to the avidin plates. After washing, the wells were incubated with 1 ug/ml of biotin-anti-TNF alpha antibody. The plate was washed and different concentrations of recombinant TNF alpha and the second antibody used as tracer were added as in Example 1 above. The plate was incubated for two hours, then washed and the alkaline phosphatase substrate was added. After 30 min the reaction was stopped and the absorbance was measured. A very good signal was obtained suggesting the absence of steric hindrances as shown in Fig. 3 herein.

### EXAMPLE 3

### Detection of TNF alpha and IFN gamma in supernatant after T-cell activation.

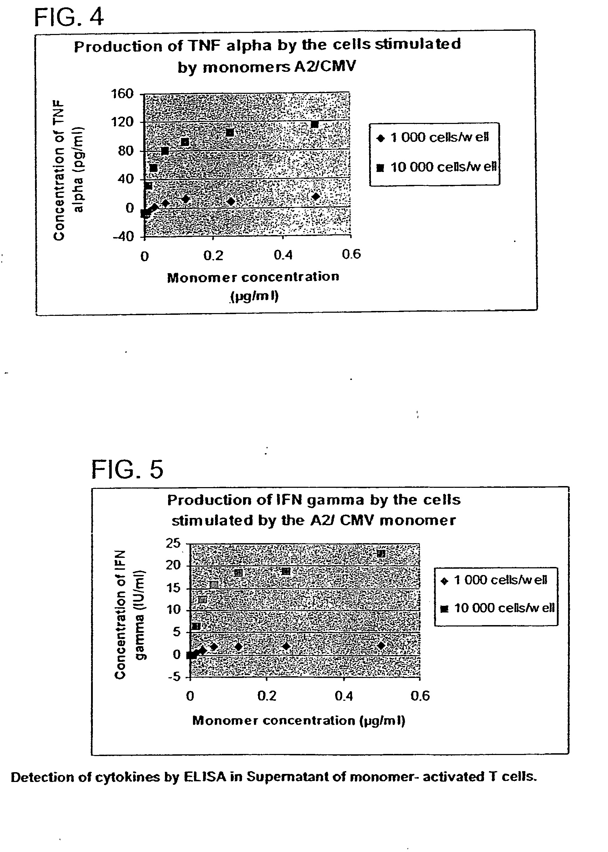

To determine whether the monomer coated plates are able to activate specifically T-cells and whether it is possible to detect cytokine release in principle, a HLA-A*0201-CMVpp65N9V-restricted polyclonal T-cell line was used. Different numbers of cells were incubated in monomer-coated plates and the cytokine release (IFN gamma and TNF alpha) after overnight incubation was measured in the supernatant by ELISA (Fig. 4). The data show that IFN gamma and TNF alpha can be detected by ELISA when the number of specific cells in the well is at least 1000 cells.

It was also observed that T-cells stimulated with the appropriate monomer (HLA-A*0201-CMVpp65 N9V) were able to secrete the IFN gamma as well as the TNF alpha in a dose response manner (Fig. 5). The level of the cytokine production was a function of the number of cells and the concentration of the monomer added into the wells.

### EXAMPLE 4

### Capture assay.

This experiment illustrates that cytokines secreted by the same polyclonal cell line specific for HLA-A*0201 CMVpp65 N9V as used in Example 3 could be detected by a capture assay. Biotinylated monomer and biotin-anti-TNF alpha coated plates were incubated overnight with the polyclonal T-cell line and secreted TNF alpha was measured after capture by the plate coated anti-TNF alpha antibodies instead of measuring TNF alpha in the supernatant by ELISA. In this experiment, after incubation overnight, the plate was washed and incubated immediately with the second tagged monoclonal antibody. Results (shown in Fig. 6) demonstrate that the assay works nicely because a clear dose response curve was obtained. A high concentration of TNF alpha when compared with the TNF alpha measured by ELISA was observed, suggesting that the capture assay is more sensitive than the ELISA. In addition a high level of TNF alpha was detected by using the capture assay with concentrations of monomer less than 0.007 µg/ml, showing that the assay is easily feasible at the monomer concentrations usually coated in standard assays. Fig. 7 shows a comparison of results obtained from cytokine measurements by ELISA and by the invention capture assay.

### EXAMPLE 5

This experiment illustrates use of MHC monomers or multimers (including tetramers) to discover epitopes recognized by patients' T-cells in response to infectious agents (e.g., CMV), cancer (e.g., melanoma) or their components in an overnight assay. For this purpose the bioassay was used to detect the activation of peptide-specific T-cells in a heterogeneous population.

Since the ultimate utility of this bioassay is to discover epitopes in a peptide pool that are capable of stimulating T-cells in patients' samples, in the experiments described below, peptide exchange was performed to replace the Mart-1 (low affinity) peptide with a CMV peptide, which serves as a model peptide in these experiments.

A. To address whether activation of peptide-specific T-cells can be detected in an overnight assay, T-cell activation was assessed by measuring CD25 (IL2 receptor) expression on T-cells. The assay is conveniently performed with flow cytometry analysis. A T-cell line was used in the assay that is enriched for T-cells specific for the peptide NLVPMVATV (also called N9V)(SEQ ID NO: 1) (59.9% of its CD8⁺ T-cells stained with a A2-CMV tetramer that was folded with this peptide).

For peptide exchange, biotinylated Mart-1 (26-35) monomers were mixed with A2-CMV peptides that were 10X or 100X in molar ratio to Mait-1 monomers. After overnight incubation at room temperature (with mild shaking), mixtures, in various dilutions, were added to streptavidin-coated (96-plate) wells. These wells were labeled as peptide-exchanged wells. Replicate wells were set up so that analysis could be done on Day I and Day 12 (See below). As positive ("+") controls, A2-CMV monomers were added to wells in a labeled plate, and as negative ("-") controls, A2-Mart 1 monomers were added to wells in a labeled plate. Plates were incubated for one hr, washed to remove the unbound material, and T-cells were then added to the wells. Plates were incubated overnight in a CO₂ incubator at 37°C, after which cells were harvested from one set of replica wells to analyze for CD25 expression (Day 1 data). The other set of replica wells were left to culture until Day 12, at which time the monomers were incorporated into monomers after incubation of the monomers for 12 days and cells were harvested to analyze for tetramer binding using flow cytometry (Day 12 data).

**Table 1 (Day 1 data)**

| Percentage of CD25+ cells in CD8+ T-cells | | | |
|---|---|---|---|
| Wells coated with | | | |
| CMV monomer "+" control | Mart-1 monomer "-" control | 10X Peptide Exchanged monomer | 100X Peptide Exchanged monomer |
| 60.3 | 12.3 | 62.0 | 58.4 |

### Table 1 (Day 1 data):

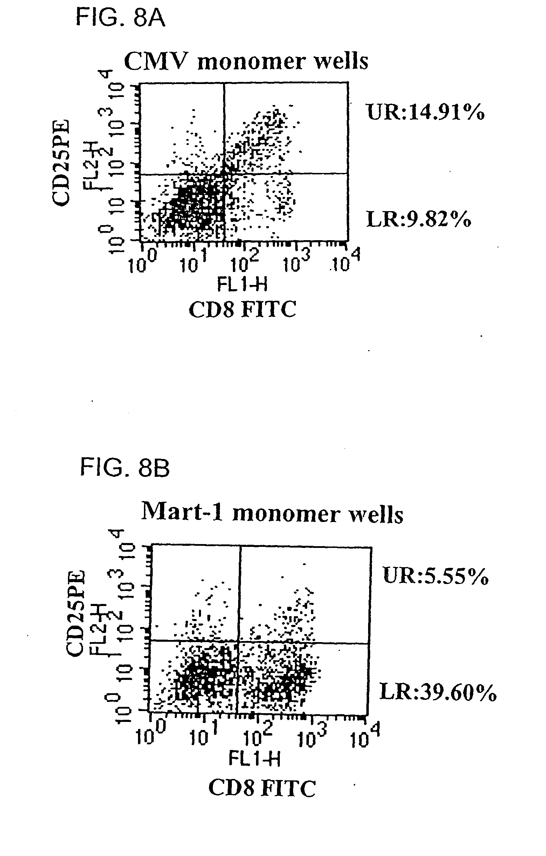

In the positive wells (wells coated with A2-CMV monomers), 60.3% of CD8+ T-cells expressed the activation marker CD25, showing 60.3% of CD8+ T-cells in this T-cell line bound A2-CMV monomers coated in the well. Density plots of these data are shown in Fig. 8A. As shown, 24.74% cells were CD8+, 14.91%, thus, 60.3% of CD8+ cells expressed CD25 in this culture.

In the negative wells (well coated with A2-Mart-1 monomers), 12.3% of CD8⁺ T-cells were activated, showing 12.3% of CD8+ T-cells in this T-cell line bound A2-Mart-1 monomers coated in the well (Fig. 8B). It should be noted that in the general population, there is a high frequency of Mart-1 reactive T-cells in the blood. Therefore, it is reasonable to find Mart-1 reactive T-cells in the test T-cell line which was derived from PBMC.

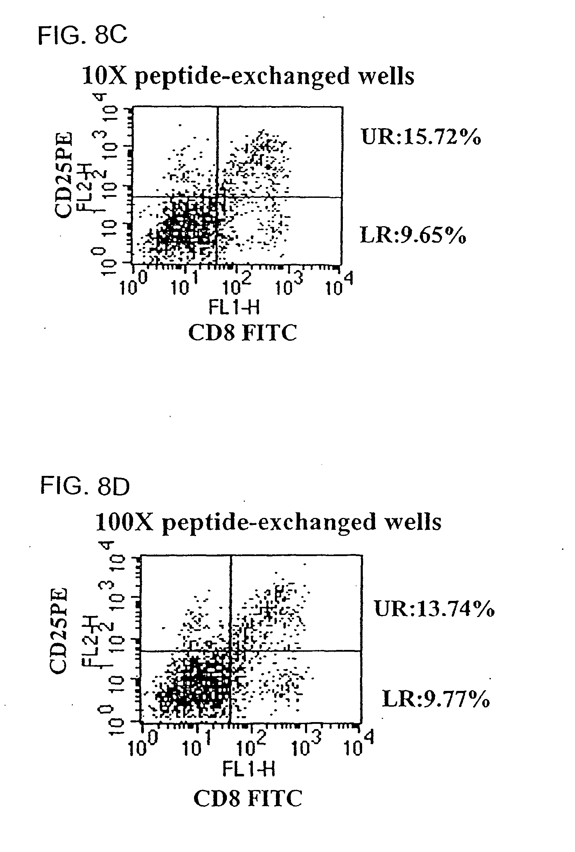

Data from wells coated with exchanged monomers (Figs. 8C and 8D) clearly demonstrated that the majority of monomers (at least 79% to 80%) in these wells replaced the Mart-1 peptide with the (exchanged) A2-CMV peptide.

These data, therefore, clearly demonstrated successful exchange of the peptide in the Mart-1 monomer with the CMV peptide. In addition, CMV-peptide reactive T-cells were detected by their expression of CD25 activation marker after overnight stimulation.

**Table 2 (Day 12 data)**

| Percentage of AZ-CMV tetramer+ cells in CD8+ T-cells | | | |
|---|---|---|---|
| Wells coated with | | | |
| CMV monomer "+" control | Mart-1 monomer "-" control | 10X Peptide Exchanged monomer | 100X Peptide Exchanged monomer |
| 89.1 | 5.0 | 93.63 | 92.16 |

### Table 2 (Day 12 data):

The purpose of carrying the T-cells out to day 12 was to confirm, by tetramer staining, that the cells activated by monomers to express CD25 (as detected on Day 1), were peptide-specific. (It should be noted that tetramer staining was not possible on Day 1 due to TCR down regulation.)

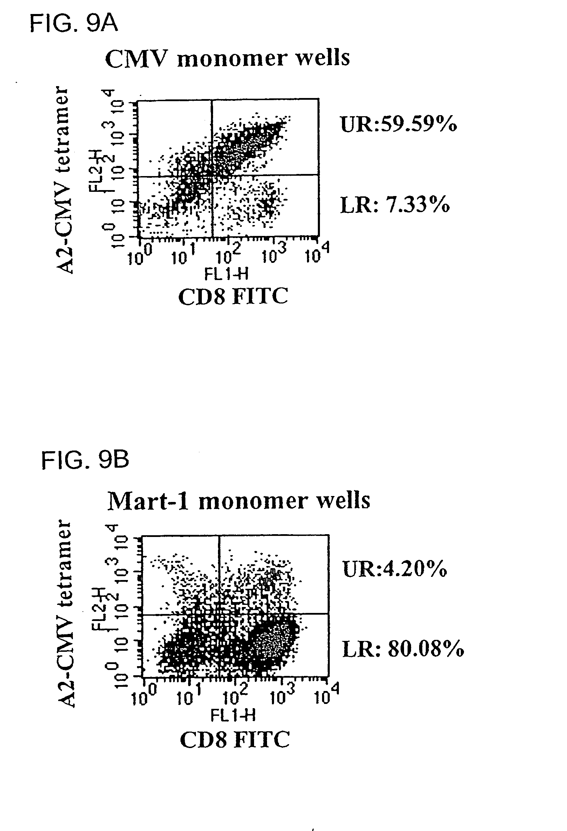

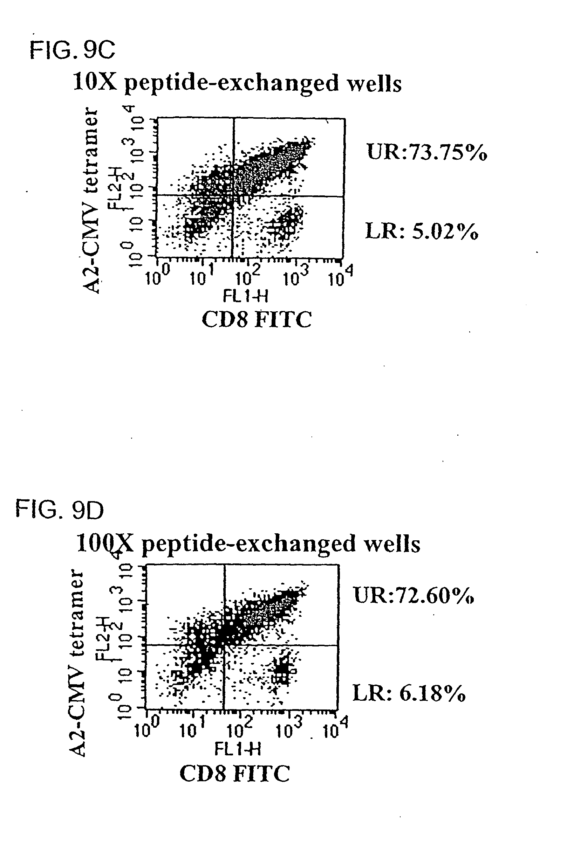

Data on Day 12 again clearly demonstrated by results of the tetramer staining assay that the majority of monomers in wells coated with exchanged monomers contained the A2-CMV peptide instead of the Mart-1 peptide. Density plots of these data are shown in Figs. 9A-D.

This experiment illustrates detection in PBMC of CMV-monomer specific activation of T-cells in peptide-exchanged wells, by measuring CD25 expression and shows that T-cells expanded out of peptide-exchanged wells contain more A2-CMV tetramer⁺ cells than those expanded from unexchanged wells (containing A2 Mart- 1 monomers).

B. The experiment was conducted using the protocol of section A above, except that PBMC instead of the known T-cell line was added to the wells. In this specific experiment, the PBMCs were enriched for CD8⁺ T-cells using magnetic beads coated with anti-CD4 Ab before plating in wells. In addition, the cells were cultured for two days instead of one day before they were assayed for CD25 expression.

**Table 3 (Day 2 data)**

| Percentage of CD25+ cells in CD8+ T-cells | | | |
|---|---|---|---|
| Wells coated with | | | |
| CMV monomer "+" control | Mart-1 monomer "-" control | 10X Peptide Exchanged monomer | 100X Peptide Exchanged monomer |
| 1.83 | 1.52 | 1.99 | 1.94 |

### Table 3 (Day 2 data):

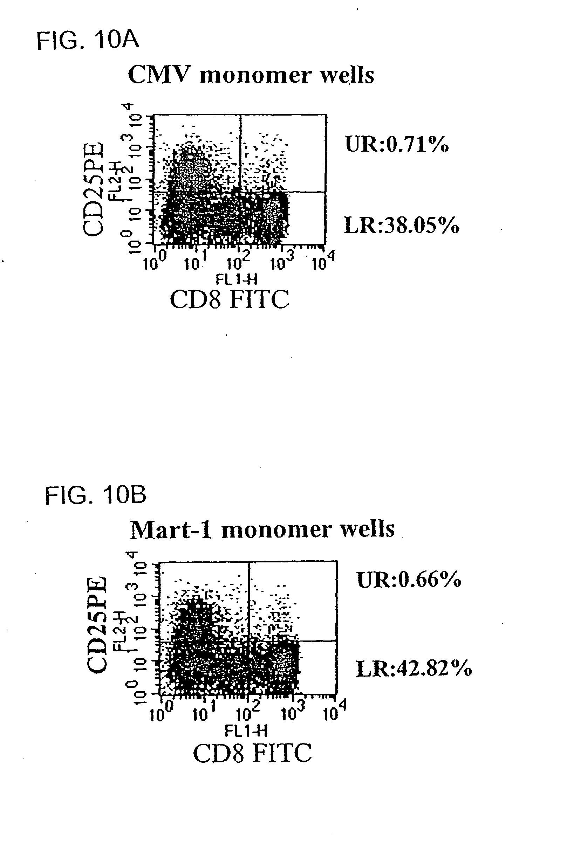

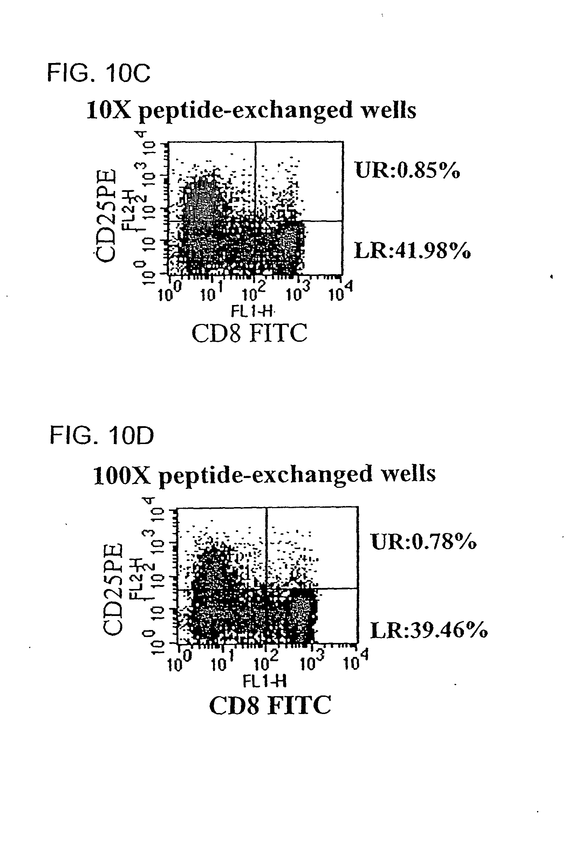

These results show that the percentage of activated T-cells from wells coated with exchanged monomers was higher than from wells containing unexchanged monomers and slightly higher than from wells coated with CMV monomers. However, this experiment addresses rare events. Therefore, although 0.47% (1.99 - 1.52) is a small number, the result is biologically significant, as supported by data obtained on Day 12 (Table 4). Density plots of these data are shown in Figs. 10A-D.

The higher percentage of activated T-cells in peptide-exchanged wells as compared to wells containing "+" control could result from some Mart-1 monomers remaining in the peptide-exchanged wells. In this case, some Mart-1 specific T-cells in PBMC, were also activated in addition to CMV-specific T-cells. As mentioned before, in the general population, there is a high frequency of Mart-1 specific T-cells in the peripheral blood.

**Table 4 (Day 12) data**

| Percentage of tetramer cells in CD8+ T-cells | | | | |
|---|---|---|---|---|
| Staining with Tetramers of | Wells coated with | | | |
| | CMV monomer "+" control | Mart-1 monomer "-" control | 10X Peptide Exchanged monomer | 100X Peptide Exchanged monomer |
| A2-CMV | 11.8 | 1.05 | 7.24 | 8.27 |
| A2 Mart-1 | 0.644 | 0.94 | 0.67 | 1.19 |

### Table 4 (Day 12 data):

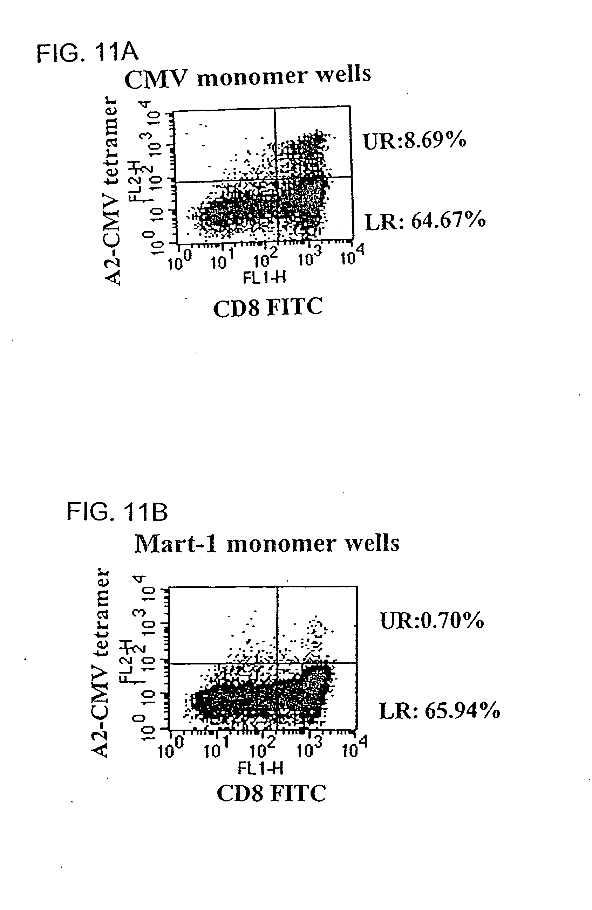

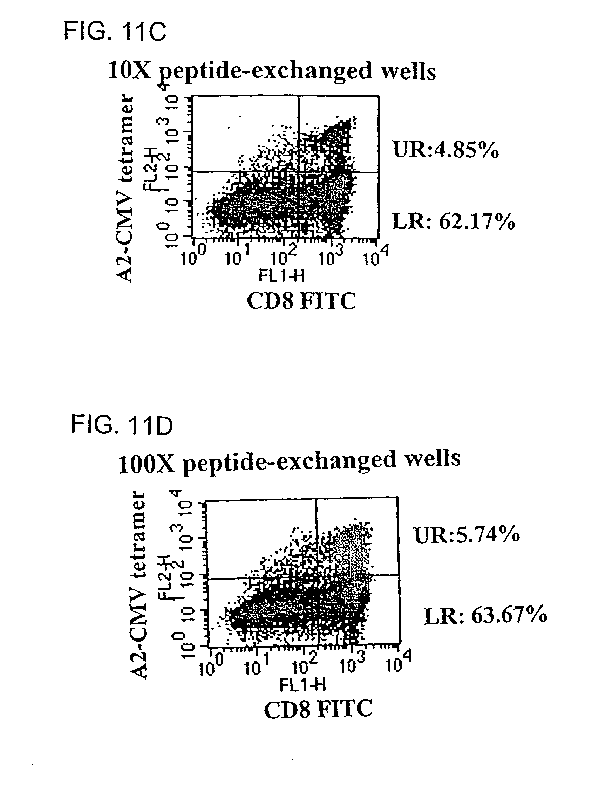

Incubation of T-cells was carried out to day 12 to confirm by tetramer staining that the cells that were activated by monomer to express CD25 (detected on Day 2), were peptide-specific. The results of the tetramer staining assay clearly shows that in wells coated with exchanged monomers the majority of cells that expanded were A2-CMV peptide-specific. These data thus demonstrate that peptide exchange was successfully carried out in this experiment, and that exchanged monomers were able to expand A2-CMV peptide-specific T-cells in culture. Density plots of these data are shown in Figs. 11 A-D.

### Flow chart for Discovery of antigenic epitopes that stimulate CD8⁺ or CD4⁺ T-cells

1. Mix A2-Mart-1 monomers with 10X molar excess of A2-CMV peptides.

2. Incubate overnight at room temperature, with shaking, in dark.

3. Add serial dilutions of this mix to avidin-coated wells in 96-well plates.

4. Also prepare "+" control wells with A2-CMV monomers; and "-"control wells with A2-Mart-1 monomers.

5. Add to these wells cell populations (PBMC or cell line) that contain A2-CMV tetramer binding T-cells.

6. Incubate overnight.

7. Harvest a portion of cells from each group to assess activation, such as CD69, CD25 expression and/or IFNγ secretion.

8. Add irradiated EBV-B cells or PBMC to the remaining cells in culture to allow proliferation/expansion. These cultures will be harvested later to stain with A2-CMV tetramers and analyzed by flow cytometry.

These results show that the invention methods can be used to discover epitopes for cancer and infectious agents, for example, by using a peptide library or cancer cell lysates in conjunction with PBMCs from patients having or suspected of having the cancer.

### EXAMPLE 6

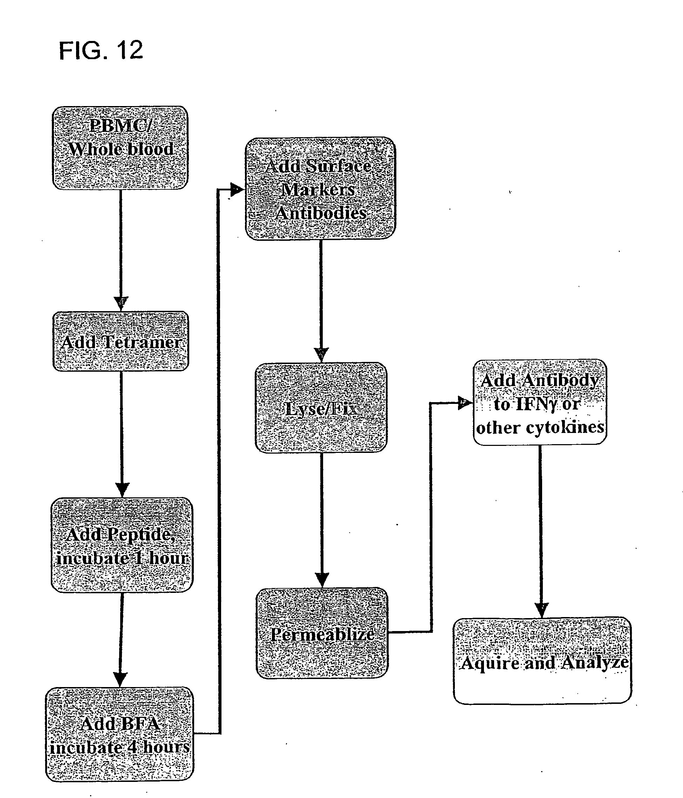

This experiment illustrates use of MHC tetramer as reagent for both stimulating and staining to enumerate (1) total tetramer-positive cells, and (2) functional tetramer-positive cells (e.g., cytokine-producing) incubated in the same tube. Human peripheral blood mononuclear cells (PBMCs) in medium or whole blood that contain A2-CMV binding, CD8⁺ cells were incubated for 30 min at room temperature with PE-labeled A2-CMV binding modified MHC tetramers, to allow for binding of tetramers to the A2-CMV binding, CD8⁺ cells in the PBMCs or whole blood (WB). Cells were further incubated without peptides at 37° C for 5 hours before being removed for use in an intracellular antibody staining assay for production of gamma-interferon (IFNγ) (Fig. 12).

FITC-labeled anti-IFNγ and PC5-labeled anti-CD8 antibodies were added and incubated for 15 minutes at room temperature, to the PBMCs or WB. A Lyse/Fix mixture was added and incubated for 10 minutes at room temperature to the PBMCs or WB (red blood cells would be lysed in this step). A fixative reagent was added to the PBMCs or lysed WB, and incubated for 10 minutes at room temperature. A permeablization reagent was added to the fixed PBMCs or lysed WB and incubated for 5 minutes at room temperature, after which anti-IFNγ was added and incubated for 30 minutes. The cells were fixed and analysed for Tetramer and IFNγ.

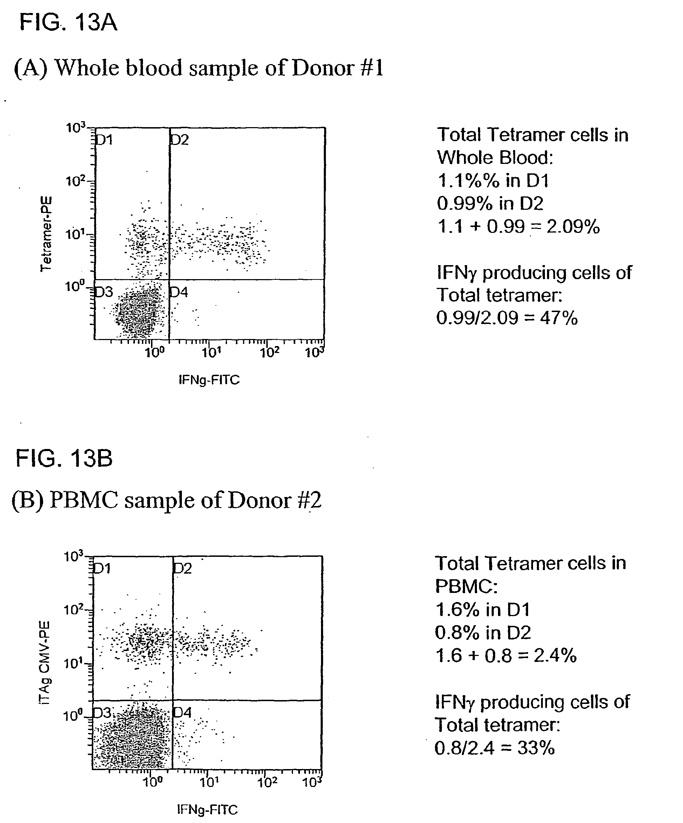

Results of this experiment (Fig. 13) clearly demonstrated: (1) A high percent of 47% of A2-CMV positive cells in whole blood experiment and 33% of A2-CMV positive cells in PBMC experiment were found to produce IFNγ; (2) CD8⁺cells exhibited a "cluster" pattern of staining, even though they were activated, as evidenced by detected production of INFγ. From these results it was concluded that (1) Engagement of T-cell receptors (TCR) with tetramers followed by peptides overcomes the threshold needed for T-cell activation; (2) activation of T-cells results in intemalisation of TCR, including the bound tetramers; (3) internalised tetramers allow the detection of tetramer⁺ cells by flow cytometry.

The high percent of IFNγ-secreting cells detected in the tetramer⁺ population shows a very efficient stimulation of T-cells by tetramers. The use of tetramers both as the staining and the stimulating reagent in some cases negates the need of using peptides in the functional assessment of tetramer⁺ cells. As tetramers (and other MHC multimers) contain the MHC elements as well as the peptides, use of tetramers as both the staining and the stimulating reagent in the same vessel (i.e., tube) makes a sensitive, reproducible assay for the measurement of functional immunity. (Figs. 12 and 13).

## Claims

1. A method of identifying a peptide capable of activating a peptide-specific T-cell, said method comprising:
a) incubating under suitable conditions peptide-specific T-cells and a solid surface having attached thereto:
1) BSA-biotin-avidin coated onto the solid surface;
2) a plurality of biotinylated MHC monomers or modified biotinylated MHC monomers having a bound candidate MHC-binding peptide wherein the biotinylated MHC monomers or modified biotinylated MHC monomers are bound via the biotin to the avidin; and
3) a biotinylated anti-TNF alpha or anti-IFN gamma antibody that forms an antibody-TNF alpha or -IFN gamma complex upon activation of the peptide-specific T-cells by the biotinylated MHC monomer - MHC-binding peptide complex; and
b) contacting the antibody-TNF alpha or IFN gamma complex obtained from a) with a second antibody specific for TNF alpha or IFN gamma and wherein the second antibody is tagged with a detectable label so as to allow formation of an antibody-TNF alpha or -IFN gamma-antibody complex; and
c) detecting a signal produced by the detectable label, wherein the detecting indicates activation of the peptide-specific T-cells and wherein the activation of the peptide-specific T cells indicates that the bound candidate MHC-binding peptide is capable of activating the peptide-specific T-cells.

2. The method of claim 1, wherein the first antibody is attached to the solid surface via binding to the avidin.

3. The method of claim 1, wherein the biotinylated MHC monomer is contained in an MHC multimer.

4. The method of claim 1, wherein the MHC binding peptide is disassociated from the MHC monomer and replaced with another MHC biding peptide.

5. The method of claim 1, wherein the first and second antibodies bind to different epitopes of the TNF alpha or IFN gamma.

6. The method of claim 1, wherein the monomers are HLA Class I.

7. The method of claim 6, wherein the peptide-specific T-cell is contained in a patient sample and the method further comprises enriching the sample for the HLA Class I monomers prior to the incubation.

8. The method of claim 6, wherein the monomers are HLA subclass A, B or C.

9. The method of claim 1, wherein the first and second antibodies are monoclonal antibodies.

10. The method of claim 1, wherein the detectable label is an enzyme, the method further comprises reacting a substrate with the enzyme, and the signal is a reaction product of the enzyme and the substrate.

11. The method of claim 10, wherein the solid surface is the wells of a microtiter plate or beads and the determining includes reading the change in color with a photometer.

12. The method of claim 1, wherein the detectable label is fluorescent.

13. The method of claim 12, wherein the detecting further comprises detecting the fluorescence using high throughput scanning and/or flow cytometry techniques.

14. The method of claim 1, wherein attachment of the monomers to the solid surface is reversible or cleavable.

15. The method of claim 1, wherein magnitude of the signal is determined and is proportional to the number of the T-cells activated.

16. The method of claim 1, wherein the peptide-specific T-cells are contained in a sample comprising a heterogeneous mixture of T-cells and wherein magnitude of the signal is determined, thereby determining a proportion of the peptide-specific T-cells contained in the sample.

17. A kit comprising: a solid surface, wherein the surface has attached thereto:
a) BSA-biotin-avidin;
b) one or more biotinylated MHC monomers, modified biotinylated MHC monomers or biotinylated multimers thereof wherein the monomers contain a suitable MHC-binding peptide and wherein the biotinylated MHC monomers, modified biotinylated MHC monomers or biotinylated multimers thereof are bound via the biotin to the avidin; and
c) a biotinylated anti-TNF alpha or anti-IFN gamma antibody.

18. The kit of claim 17, wherein the solid surface is a bead.

19. The kit of claim 17, wherein the solid surface is in a microtiter plate.

20. The kit of claim 17 further comprising a control peptide to which the MHC monomer binds in a reconstituted form.

21. The kit of claim 17, wherein the kit further contains an additional antibody specific for TNF alpha or IFN gamma.

## Patentansprüche

1. Verfahren zum Identifizieren eines Peptids, das befähigt ist, eine Peptid-spezifische T-Zelle zu aktivieren, wobei das Verfahren umfaßt:
a) Inkubieren Peptid-spezifischer T-Zellen und einer festen Oberfläche, die darin gebunden aufweist:
1) BSA-Biotin-Avidin, das auf die feste Oberfläche beschichtet ist,
2) eine Vielzahl von biotinylierten MHC-Monomeren oder modifizierten biotinylierten MHC-Monomeren, die ein gebundenes MHC-bindendes Kandidaten-Peptid aufweisen, wobei die biotinylierten MHC-Monomere oder modifizierten biotinylierten MHC-Monomere über das Biotin an das Avidin gebunden sind, und
3) einen biotinylierten anti-TNF alpha- oder anti-IFN gamma-Antikörper, der bei Aktivierung der Peptid-spezifischen T-Zellen durch den biotinylierten MHC-Monomer-MHC-bindendes Peptid-Komplex einen Antikörper-TNF alpha- oder -IFN gamma-Komplex bildet,
unter geeigneten Bedingungen, und
b) Inkontaktbringen des Antikörper-TNF alpha- oder -IFN gamma-Komplexes, der aus a) erhalten wurde, mit einem zweiten Antikörper, der spezifisch für TNF alpha oder IFN gamma ist, und wobei der zweite Antikörper mit einer detektierbaren Markierung markiert ist, so daß die Bildung eines Antikörper-TNF alpha- oder -IFN gamma-Antikörper-Komplexes ermöglicht wird, und
c) Nachweisen eines Signals, das von der detektierbaren Markierung erzeugt wird, wobei das Nachweisen die Aktivierung der Peptid-spezifischen T-Zellen anzeigt, und wobei die Aktivierung der Peptid-spezifischen T-Zellen anzeigt, daß das gebundene MHC-bindende Kandidaten-Peptid befähigt ist, die Peptid-spezifischen T-Zellen zu aktivieren.

2. Verfahren nach Anspruch 1, wobei der erste Antikörper über Bindung an das Avidin an der festen Oberfläche gebunden ist.

3. Verfahren nach Anspruch 1, wobei das biotinylierte MHC-Monomer in einem MHC-Multimer enthalten ist.

4. Verfahren nach Anspruch 1, wobei das MHC-bindende Peptid von dem MHC-Monomer dissoziiert wird und durch ein anderes MHC-bindendes Peptid ersetzt wird.

5. Verfahren nach Anspruch 1, wobei die ersten und zweiten Antikörper verschiedene Epitope des TNF alpha oder IFN gamma binden.

6. Verfahren nach Anspruch 1, wobei die Monomere HLA-Klasse I sind.

7. Verfahren nach Anspruch 6, wobei die Peptid-spezifische T-Zelle in einer Patientenprobe enthalten ist und das Verfahren weiter das Anreichern der Probe auf HLA-Klasse I-Monomere vor der Inkubation umfaßt.

8. Verfahren nach Anspruch 6, wobei die Monomere HLA-Unterklassen A, B oder C sind.

9. Verfahren nach Anspruch 1, wobei die ersten und zweiten Antikörper monoklonale Antikörper sind.

10. Verfahren nach Anspruch 1, wobei die nachweisbare Markierung ein Enzym ist, das Verfahren weiter das Umsetzen eines Substrats mit dem Enzym umfaßt und das Signal ein Reaktionsprodukt des Enzyms und des Substrats ist.

11. Verfahren nach Anspruch 10, wobei die feste Oberfläche die Vertiefungen einer Mikrotiterplatte oder Perlen (*beads*) ist, und das Bestimmen das Auslesen der Änderung in der Farbe mit einem Photometer einschließt.

12. Verfahren nach Anspruch 1, wobei die nachweisbare Markierung ein fluoreszierender Stoff ist.

13. Verfahren nach Anspruch 12, wobei das Nachweisen weiter das Nachweisen der Fluoreszenz unter Verwendung von Hochdurchsatz-Scannen und/oder Durchflußcytometrie-Techniken umfaßt.

14. Verfahren nach Anspruch 1, wobei Bindung der Monomere an die feste Oberfläche reversibel oder spaltbar ist.

15. Verfahren nach Anspruch 1, wobei die Größe des Signals bestimmt wird und diese proportional zu der Anzahl der aktivierten T-Zellen ist.

16. Verfahren nach Anspruch 1, wobei die Peptid-spezifischen T-Zellen in einer Probe enthalten sind, umfassend eine heterogene Mischung von T-Zellen, und wobei die Größe des Signals bestimmt wird, wodurch ein Teil der Peptid-spezifischen T-Zellen, die in der Probe enthalten sind, bestimmt wird.

17. Kit, umfassend: eine feste Oberfläche, wobei die feste Oberfläche daran gebunden aufweist:
a) BSA-Biotin-Avidin,
b) ein oder mehrere biotinylierte MHC-Monomere, modifizierte biotinylierte MHC-Monomere oder biotinylierte Multimere davon, wobei die Monomere ein geeignetes MHC-bindendes Peptid enthalten, und wobei die biotinylierten MHC-Monomere, modifizierten biotinylierten MCH-Monomere oder biotinylierten Multimere davon über das Biotin an das Avidin gebunden sind, und
c) einen biotinylierten anti-TNF alpha- oder anti-IFN gamma-Antikörper.

18. Kit nach Anspruch 17, wobei die feste Oberfläche eine Perle bzw. Bead ist.

19. Kit nach Anspruch 17, wobei die feste Oberfläche eine Mikrotiterplatte ist.

20. Kit nach Anspruch 17, weiter umfassend ein Kontrollpeptid, an welches das MHC-Monomer in einer rekonstituierten Form bindet.

21. Kit nach Anspruch 17, wobei das Kit weiter einen zusätzlichen Antikörper, der spezifisch für TNF alpha oder IFN gamma ist, enthält.

## Revendications

1. Procédé pour identifier un peptide capable d'activer une cellule T spécifique à un peptide, ledit procédé consistant à :
a) incuber dans des conditions appropriées des cellules T spécifiques à un peptide et une surface solide à laquelle sont attachés :
1) du BSA-biotine-avidine recouvrant la surface solide ;
2) une pluralité de monomères du CMH biotinylés ou de monomères du CMH biotinylés modifiés auxquels est lié un peptide de liaison au CMH candidat, où les monomères du CMH biotinylés ou monomères du CMH biotinylés modifiés sont liés via la biotine à l'avidine ; et
3) un anticorps anti-TNF alpha ou anti-IFN gamma biotinylé qui forme un complexe anticorps-TNF alpha ou -IFN gamma suite à une activation des cellules T spécifiques à un peptide par le complexe monomère du CMH biotinylé-peptide de liaison au CMH ; et
b) mettre en contact le complexe anticorps-TNF alpha ou IFN gamma obtenu en a) avec un second anticorps spécifique pour le TNF alpha ou l'IFN gamma, et où le second anticorps est marqué avec un marqueur détectable de sorte à permettre la formation d'un complexe anticorps-TNF alpha ou -IFN gamma-anticorps ; et
c) détecter un signal produit par le marqueur détectable, où la détection indique une activation des cellules T spécifiques à un peptide, et où l'activation des cellules T spécifiques à un peptide indique que le peptide de liaison au CMH candidat lié est capable d'activer les cellules T spécifiques à un peptide.

2. Procédé selon la revendication 1, dans lequel le premier anticorps est attaché à la surface solide via une liaison à l'avidine.

3. Procédé selon la revendication 1, dans lequel le monomère du CMH biotinylé est contenu dans un multimère du CMH.

4. Procédé selon la revendication 1, dans lequel le peptide de liaison au CMH est dissocié du monomère du CMH et remplacé par un autre peptide de liaison au CMH.

5. Procédé selon la revendication 1, dans lequel le premier et le second anticorps se lient à différents épitopes du TNF alpha ou de l'IFN gamma.

6. Procédé selon la revendication 1, dans lequel les monomères sont des molécules HLA de classe 1.

7. Procédé selon la revendication 6, dans lequel la cellule T spécifique à un peptide est contenue dans un échantillon d'un patient et le procédé consiste en outre à enrichir l'échantillon en monomères HLA de classe I avant l'incubation.

8. Procédé selon la revendication 6, dans lequel les monomères sont HLA sous-classe A, B ou C.

9. Procédé selon la revendication 1, dans lequel le premier et le second anticorps sont des anticorps monoclonaux.

10. Procédé selon la revendication 1, dans lequel le marqueur détectable est une enzyme, le procédé consistant en outre à faire réagir un substrat avec l'enzyme, et le signal est un produit de réaction de l'enzyme et du substrat.

11. Procédé selon la revendication 10, dans lequel la surface solide consiste en les puits d'une plaque de microtitration ou des billes et la détermination consiste à lire le changement de couleur avec un photomètre.

12. Procédé selon la revendication 1, dans lequel le marqueur détectable est fluorescent.

13. Procédé selon la revendication 12, dans lequel la détection consiste en outre à détecter la fluorescence en utilisant des techniques à haut débit de criblage et/ou de cytométrie en flux.

14. Procédé selon la revendication 1, dans lequel l'attachement des monomères à la surface solide est réversible ou clivable.

15. Procédé selon la revendication 1, dans lequel la magnitude du signal est déterminée et est proportionnelle au nombre des cellules T activées.

16. Procédé selon la revendication 1, dans lequel les cellules T spécifiques à un peptide sont contenues dans un échantillon comprenant un mélange hétérogène de cellules T et dans lequel la magnitude du signal est déterminée, ce qui permet ainsi de déterminer une proportion des cellules T spécifiques à un peptide contenues dans l'échantillon.

17. Trousse comprenant : une surface solide, surface à laquelle sont attachés :
a) du BSA-biotine-avidine ;
b) un ou plusieurs monomères du CMH biotinylés, des monomères du CMH biotinylés modifiés ou des multimères biotinylés de ceux-ci, où les monomères contiennent un peptide de liaison au CMH approprié et où les monomères du CMH biotinylés, les monomères du CMH biotinylés modifiés ou les multimères biotinylés de ceux-ci sont liés via la biotine à l' avidine ; et
c) un anticorps anti-TNF alpha ou anti-IFN gamma biotinylé.

18. Trousse selon la revendication 17, où la surface solide est une bille.

19. Trousse selon la revendication 17, où la surface solide est dans une plaque de microtitration.

20. Trousse selon la revendication 17, comprenant en outre un peptide de contrôle auquel se lie le monomère du CMH sous une forme reconstituée.

21. Trousse selon la revendication 17, où le trousse contient également un anticorps supplémentaire qui est spécifique pour le TNF alpha ou l'IFN gamma.
